# EUROPÄISCHE PATENTSCHRIFT

(11) **EP 3 821 785 B1**
(45) Veröffentlichungstag und Bekanntmachung des Hinweises auf die Patenterteilung: **09.08.2023**
(21) Anmeldenummer: 20206956.3
(22) Anmeldetag: 11.11.2020
(51) Int. Cl.: A61B 1/00, G02B 23/24

(54) **STEREO-ENDOSKOP**
STEREO ENDOSCOPE
ENDOSCOPE STÉRÉO

(30) Priorität: 13.11.2019 DE 102019130593
(43) Veröffentlichungstag der Anmeldung: 19.05.2021
(73) Patentinhaber: Karl Storz SE & Co. KG, 78532 Tuttlingen (DE)
(72) Erfinder: Forster, Jonas, 78532 Tuttlingen (DE)

(56) Entgegenhaltungen:
- WO-A1-2019/072685
- US-A1- 2013 038 689
- US-A1- 2013 041 226

## Beschreibung

Die vorliegende Erfindung ist auf ein Stereo-Endoskop zum Erfassen eines für die Betrachtung mit dem linken Auge vorgesehenen Bilds und eines für die Betrachtung mit dem rechten Auge vorgesehenen Bilds bezogen. Eine Ausführungsform der Erfindung ist insbesondere auf ein Stereo-Endoskop bezogen, bei dem die Bildsensoren zum Erfassen der beiden Bilder in einem distalen Endbereich des Stereo-Endoskops angeordnet sind.

Bei einem Endoskop kann das durch ein Objektiv am distalen Ende erzeugte Bild durch ein Relaislinsensystem, ein geordnetes Bündel von Lichtleitfasern oder auf andere Weise zum proximalen Ende übertragen und dort durch eine integrierte oder mechanisch und optisch angekoppelte Kamera erfasst und ein entsprechendes Bildsignal erzeugt werden. Alternativ kann das durch das Objektiv erzeugte Bild durch einen nahe dem distalen Ende angeordneten Bildsensor erfasst und ein entsprechendes Bildsignal erzeugt werden. Dies gilt gleichermaßen für monokulare und für stereoskopische Endoskope.

Besonders die Anordnung zweier Bildsensoren in einem distalen Endbereich eines Endoskops stellt eine technische Herausforderung dar. Diese Herausforderung wird durch die zunehmende Miniaturisierung noch größer. Es existieren mehrere Ansätze, um in einem möglichst kleinen Bauraum zwei möglichst große Bildsensoren anzuordnen.

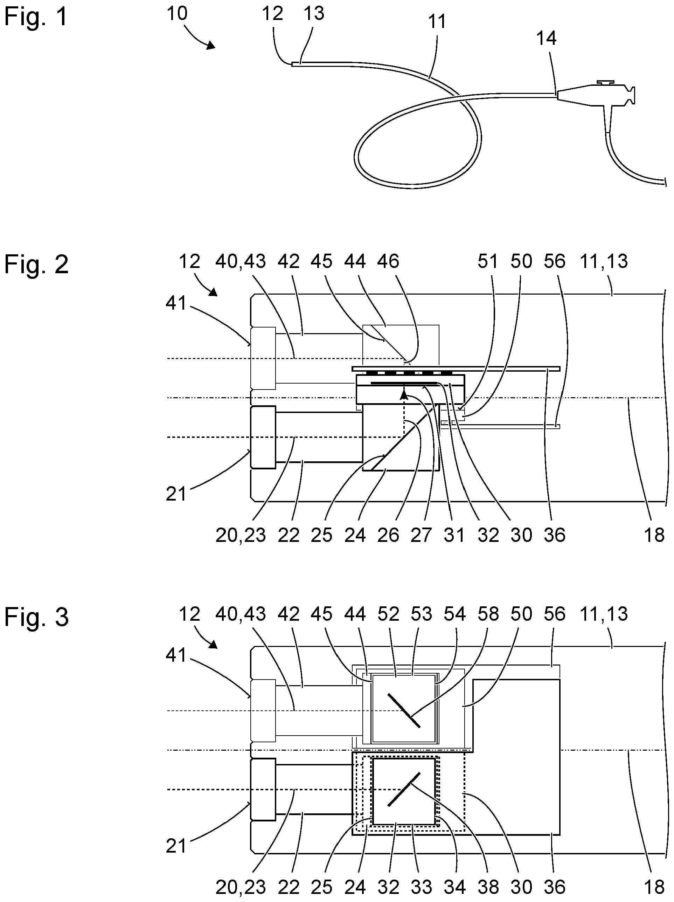

In US 4,873,572 sind mehrere elektronische Endoskope mit jeweils zwei Bildsensoren 214a, 214b; 264a, 264b; 293a, 293b oder zwei Bildgebungsbereichen 21, 22 beschrieben (Zusammenfassung; Spalte 4, Zeilen 16 bis 26; Spalte 12, Zeilen 12 bis 16; Spalte 15, Zeilen 22 bis 33; Spalte 16, Zeile 53, bis Spalte 17, Zeile 3; Figuren 1, 10, 12, 15 bis 18, 20 bis 22). Die zwei Bildsensoren 214a, 214b; 264a, 264b; 293a, 293b oder zwei Bildgebungsbereiche 21, 22 sind entweder nebeneinander oder Rücken an Rücken angeordnet.

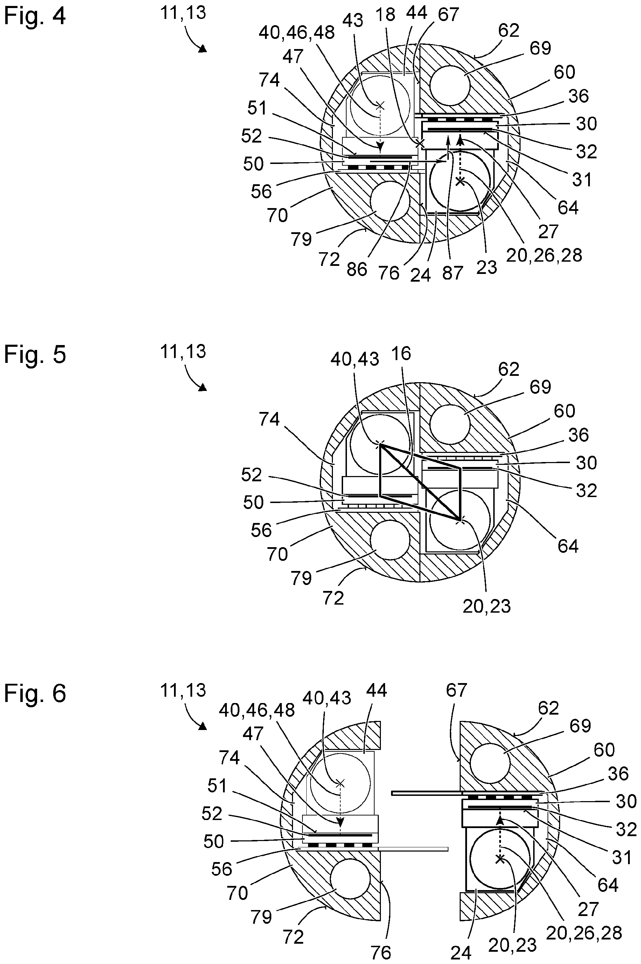

In US 5,689,365 sind Stereo-Endoskope mit jeweils zwei nebeneinander angeordneten Bildsensoren 18; 18L, 18R beschrieben (Figuren 4, 5, 7, 8, 10, 13, 17 bis 20, 23, 25, 27, 28, 32, 33).

In der DE102017123896A 1, die in Kombination die Merkmale der Oberbegriffe der Ansprüche 1 und 7 zeigt, ist ein Stereo-Endoskop offenbart, bei dem zwei Bildsensoren parallel und versetzt zueinander im Schaft angeordnet sind. Die Stereobasis des Instruments ist parallel zu den Sensoren ausgerichtet. Diese Erfindung wurde auch als Anmeldung WO2019072685A1 veröffentlicht.

Die US2018/0196251A1 offenbart ein Endoskop mit einer einzelnen Apertur, wobei einfallendes Licht im weiteren Verlauf des Strahlengangs über Prismen aufgeteilt wird, so dass zwei Bildsensoren je einen Teil des Bildes erzeugen. Die Bildsensoren sind hierzu parallel zueinander liegend im Schaft angeordnet.

Aus der US2017/0245744A1 ist ein Endoskop mit einem bildgebenden System mit zwei Strahlengängen bekannt, wobei einfallendes Licht jeweils über ein Prisma auf einen Bildsensor umgelenkt wird. Die beiden Bildsensoren sind parallel zueinander und zur Schaftachse und senkrecht zur Stereobasis angeordnet.

Eine Aufgabe der vorliegenden Erfindung besteht darin, ein verbessertes Stereo-Endoskop zu schaffen.

Diese Aufgabe wird durch die Gegenstände der unabhängigen Ansprüche gelöst. Weiterbildungen sind in den abhängigen Ansprüchen angegeben.

Ein Stereo-Endoskop zum Erfassen eines für die Betrachtung mit einem ersten Auge vorgesehenen ersten Bilds und eines für die Betrachtung mit einem zweiten Auge vorgesehenen zweiten Bilds umfasst einen Schaft mit einem distalen Endbereich, einen ersten Bildsensor in dem distalen Endbereich des Schafts zum Erfassen des ersten Bilds und einen zweiten Bildsensor in dem distalen Endbereich des Schafts zum Erfassen des zweiten Bilds, wobei der distale Endbereich des Schafts zwei gleiche oder im Wesentlichen gleiche Bauteile, die nebeneinander angeordnet und miteinander gefügt sind und einen äußeren Oberflächenbereich des distalen Endbereichs bilden, umfasst. Jedes der beiden Bauteile umfasst insbesondere einen der beiden Strahlengänge des Stereo-Endoskops.

Die beiden Bauteile weisen insbesondere jeweils eine zylindrische Gestalt und einen halbkreisförmigen oder im Wesentlichen halbkreisförmigen Querschnitt auf. Die beiden Bauteile können den gesamten distalen Endbereich oder einen Teil des distalen Endbereichs bilden. Unter einem Zylinder oder dem Begriff zylindrisch wird im Weiteren ein allgemeiner Zylinder verstanden, wie er in der Mathematik definiert ist. Die Mathematik definiert den allgemeinen Zylinder als einen geometrischen Körper, der von einer Zylinderfläche und zwei parallelen Ebenen begrenzt wird. Unter einer Zylinderfläche wird dabei eine Fläche verstanden, die aus allen Geraden g des Raumes besteht, die mit einer vorgegebenen Kurve k, der Leitkurve der Zylinderfläche, jeweils einen gemeinsamen Punkt besitzen und zu einer vorgegebenen Geraden g0, die ebenfalls k schneidet, parallel sind. Diese Geraden werden als die Erzeugenden der Zylinderfläche bezeichnet. Ein Spezialfall des allgemeinen Zylinders ist der gerade Kreiszylinder. Vorliegend ist jedoch die allgemeine Definition des Zylinders gemeint.

Ein Stereo-Endoskop zum Erfassen eines für die Betrachtung mit einem ersten Auge vorgesehenen ersten Bilds und eines für die Betrachtung mit einem zweiten Auge vorgesehenen zweiten Bilds umfasst einen Schaft mit einem distalen Endbereich, einen ersten Bildsensor in dem distalen Endbereich des Schafts zum Erfassen des ersten Bilds und einen zweiten Bildsensor in dem distalen Endbereich des Schafts zum Erfassen des zweiten Bilds, wobei sowohl die lichtempfindliche Schicht des ersten Bildsensors als auch die lichtempfindliche Schicht des zweiten Bildsensors parallel oder im Wesentlichen parallel zu einer Längsachse des distalen Endbereichs angeordnet ist, wobei der erste Bildsensor und der zweite Bildsensor in entgegengesetzte Richtungen orientiert sind, wobei der erste Bildsensor und der zweite Bildsensor in einer Richtung, die orthogonal zu einer Längsachse des distalen Endbereichs des Schafts und parallel zu zumindest entweder einer lichtempfindlichen Schicht des ersten Bildsensors oder einer lichtempfindlichen Schicht des zweiten Bildsensors ist, relativ zu einander versetzt angeordnet sind.

Das Stereo-Endoskop ist insbesondere für die Verwendung bei mikroinvasiven Maßnahmen oder für andere medizinische Zwecke vorgesehen und ausgebildet. Alternativ kann das Stereo-Endoskop für nicht-medizinische Zwecke vorgesehen und ausgebildet sein. Das Stereo-Endoskop kann einen oder mehrere Arbeitskanäle, durch die Fluide geleitet oder medizinische Instrumente geführt werden können, aufweisen oder als Trokarhülse ausgebildet sein.

Der Schaft des Stereo-Endoskops kann gerade oder gekrümmt, starr oder flexibel ausgebildet sein. Der distale Endbereich des Schafts ist insbesondere starr und gerade ausgebildet, so dass die äußere Mantelfläche des distalen Endbereichs zylindrisch ist. Der Querschnitt des distalen Endbereichs ist insbesondere kreisförmig. Die Längsachse des distalen Endbereichs ist insbesondere die Symmetrieachse, zu der die äußere Mantelfläche des distalen Endbereichs translationssymmetrisch und/oder rotationssymmetrisch ist

Der erste Bildsensor ist insbesondere zum Erfassen eines für die Betrachtung mit dem linken Auge vorgesehenen Bilds vorgesehen, wobei dieses Bild auch als linkes Bild bezeichnet wird. Der zweite Bildsensor ist insbesondere zum Erfassen eines für die Betrachtung mit dem rechten Auge vorgesehenen Bilds vorgesehen, wobei dieses Bild auch als rechtes Bild bezeichnet wird.

Jeder der beiden Bildsensoren weist einen dünnen schichtförmigen lichtempfindlichen Bilderfassungsbereich auf, der auch als lichtempfindliche Schicht bezeichnet wird. Der dünne schichtförmige Bilderfassungsbereich ist insbesondere eben und rechteckig. Der Bilderfassungsbereich jedes Bildsensors kann für verschiedene Wellenlängenbereiche empfindliche Teilbereiche innerhalb derselben oder in mehreren verschiedenen Ebenen aufweisen. Die schichtförmigen Bilderfassungsbereiche oder lichtempfindlichen Schichten jedes Bildsensors sind insbesondere parallel oder im Wesentlichen parallel zu einer Längsachse des distalen Endbereichs des Schafts des Stereo-Endoskops angeordnet.

Der schichtförmige Bilderfassungsbereich jedes Bildsensors ist insbesondere dicht unter (das heißt in Lichtausbreitungsrichtung hinter) einer Lichteintrittsfläche des Bildsensors angeordnet. Die entgegengesetzte Orientierung der Bildsensoren bedeutet insbesondere eine entgegengesetzte Orientierung der Lichteintrittsflächen der Bildsensoren. Zwei Richtungen sind entgegengesetzt, wenn sie einen Winkel von mindestens 120 Grad einschließen, insbesondere wenn sie einen Winkel von mindestens 150 Grad oder mindestens 170 Grad oder von 180 Grad einschlie-ßen.

Die in der beschriebenen Richtung versetzte Anordnung der Bildsensoren ist eine Anordnung der Bildsensoren nebeneinander oder im Wesentlichen nebeneinander. Die Anordnung der Bildsensoren im Wesentlichen nebeneinander, aber mit entgegengesetzter oder im Wesentlichen entgegengesetzter Orientierung kann eine besonders gute Ausnutzung eines vorhandenen Bauraums und damit ein besonders gutes Verhältnis zwischen der Größe der Sensoren und der Größe des benötigten Bauraums ermöglichen.

Bei einem Stereo-Endoskop, wie es hier beschrieben ist, sind eine lichtempfindliche Schicht des ersten Bildsensors und eine lichtempfindliche Schicht des zweiten Bildsensors insbesondere in einer Richtung, die orthogonal zu den lichtempfindlichen Schichten beider Bildsensoren und orthogonal zu einer Längsachse des distalen Endbereichs des Schafts ist, relativ zu einander versetzt angeordnet.

Die nicht nur in Richtung parallel zu mindestens einer lichtempfindlichen Schicht, sondern auch in Richtung orthogonal zu mindestens einer lichtempfindlichen Schicht versetzte Anordnung der Bildsensoren mit entgegengesetzter oder im Wesentlichen entgegengesetzter Orientierung kann eine besonders gute Ausnutzung eines vorhandenen Bauraums und damit ein besonders gutes Verhältnis zwischen der Größe der Sensoren und der Größe des benötigten Bauraums ermöglichen. Damit kann ein Stereo-Endoskop mit besonders kleinem Durchmesser realisiert werden.

Bei einem Stereo-Endoskop, wie es hier beschrieben ist, ist die lichtempfindliche Schicht des ersten Bildsensors relativ zu der lichtempfindlichen Schicht des zweiten Bildsensors insbesondere in Lichtausbreitungsrichtung von Licht unmittelbar vor Auftreffen auf den ersten Bildsensor versetzt angeordnet.

Alternativ kann die lichtempfindliche Schicht des ersten Bildsensors relativ zu der lichtempfindlichen Schicht des zweiten Bildsensors entgegen der Lichtausbreitungsrichtung von Licht unmittelbar vor Auftreffen auf den ersten Bildsensor versetzt angeordnet sein.

Mit der Lichtausbreitungsrichtung ist die Ausbreitungsrichtung von Licht, das von einem beobachteten Objekt ausgeht und sich entlang der optischen Achse eines zu dem jeweiligen Bildsensor führenden Strahlengangs ausbreitet, gemeint. Die optische Achse kann dabei insbesondere an reflektierenden Flächen die Richtung wechseln. Zwischen reflektierenden Flächen ist die optische Achse insbesondere gerade und entspricht beispielsweise im Falle rotationssymmetrischer Linsen deren Symmetrieachsen. Insbesondere im Falle von nicht rotationssymmetrischen Linsen kann als Lichtausbreitungsrichtung alternativ die mittlere Ausbreitungsrichtung von Licht, das von betrachteten Objekten ausgeht und zur Erzeugung eines Bilds in dem betreffenden Bildsensor beiträgt, verwendet werden.

Die Lichtausbreitungsrichtung von Licht unmittelbar vor Auftreffen des Lichts auf einen Bildsensor ist insbesondere die Lichtausbreitungsrichtung des Lichts zwischen einer letzten reflektierenden Fläche und der lichtempfindlichen Schicht des Bildsensors. Diese Lichtausbreitungsrichtung ist insbesondere orthogonal oder im Wesentlichen orthogonal zu der Längsachse des distalen Endbereichs des Schafts.

Ein Stereo-Endoskop, wie es hier beschrieben ist, umfasst insbesondere eine erste reflektierende Fläche zum Reflektieren von Licht, eine zweite reflektierende Fläche zum Reflektieren von Licht, einen ersten Strahlengang mit einem ersten geraden Abschnitt, der sich in Lichtausbreitungsrichtung bis zu der ersten reflektierenden Fläche erstreckt, und einem zweiten geraden Abschnitt, der sich von der ersten reflektierenden Fläche bis zu dem ersten Bildsensor erstreckt, und einen zweiten Strahlengang mit einem ersten geraden Abschnitt, der sich in Lichtausbreitungsrichtung bis zu der zweiten reflektierende Fläche erstreckt, und einem zweiten geraden Abschnitt, der sich von der zweiten reflektierenden Fläche bis zu dem zweiten Bildsensor erstreckt, wobei der erste gerade Abschnitt und der zweite gerade Abschnitt des ersten Strahlengangs in einer ersten Strahlengang-Ebene angeordnet sind, wobei der erste gerade Abschnitt und der zweite gerade Abschnitt des zweiten Strahlengangs in einer zweiten Strahlengang-Ebene, die von der ersten Strahlengang-Ebene verschieden ist, angeordnet ist, und wobei eine Lichtausbreitungsrichtung von Licht in dem zweiten geraden Abschnitt des ersten Strahlengangs und eine Lichtausbreitungsrichtung von Licht in dem zweiten geraden Abschnitt des zweiten Strahlengang entgegengesetzt oder im Wesentlichen entgegengesetzt sind.

Ein Stereo-Endoskop zum Erfassen eines für die Betrachtung mit einem ersten Auge vorgesehenen ersten Bilds und eines für die Betrachtung mit einem zweiten Auge vorgesehenen zweiten Bilds umfasst einen Schaft mit einem distalen Endbereich, einen ersten Bildsensor in dem distalen Endbereich des Schafts zum Erfassen des ersten Bilds, einen zweiten Bildsensor in dem distalen Endbereich des Schafts zum Erfassen des zweiten Bilds, eine erste reflektierenden Fläche zum Reflektieren von Licht, eine zweite reflektierende Fläche zum Reflektieren von Licht, einen ersten Strahlengang mit einem ersten geraden Abschnitt, der sich in Lichtausbreitungsrichtung bis zu der ersten reflektierenden Fläche erstreckt, und einem zweiten geraden Abschnitt, der sich von der ersten reflektierenden Fläche bis zu dem ersten Bildsensor erstreckt, und einen zweiten Strahlengang mit einem ersten geraden Abschnitt, der sich in Lichtausbreitungsrichtung bis zu der zweiten reflektierende Fläche erstreckt, und einem zweiten geraden Abschnitt, der sich von der zweiten reflektierenden Fläche bis zu dem zweiten Bildsensor erstreckt, wobei der erste gerade Abschnitt und der zweite gerade Abschnitt des ersten Strahlengangs in einer ersten Strahlengang-Ebene angeordnet sind, wobei der erste gerade Abschnitt und der zweite gerade Abschnitt des zweiten Strahlengangs in einer zweiten Strahlengang-Ebene, die von der ersten Strahlengang-Ebene verschieden ist, angeordnet ist, und wobei eine Lichtausbreitungsrichtung von Licht in dem zweiten geraden Abschnitt des ersten Strahlengangs und eine Lichtausbreitungsrichtung von Licht in dem zweiten geraden Abschnitt des zweiten Strahlengang entgegengesetzt oder im Wesentlichen entgegengesetzt sind. Das Stereo-Endoskop kann im Übrigen gleiche oder ähnliche Eigenschaften wie die übrigen hier beschriebenen Stereo-Endoskope aufweisen.

Die erste reflektierende Fläche ist bezogen auf die Lichtausbreitungsrichtung insbesondere in dem Sinne unmittelbar vor dem ersten Bildsensor angeordnet, dass zwischen der ersten reflektierenden Fläche und dem ersten Bildsensor keine weitere reflektierende Fläche angeordnet ist.

Zwischen der ersten reflektierenden Fläche und dem ersten Bildsensor breitet Licht sich geradlinig oder im Wesentlichen geradlinig aus, und wird insbesondere allenfalls an Grenzflächen zwischen verschiedenen Materialien gebrochen. Die zweite reflektierende Fläche ist bezogen auf die Lichtausbreitungsrichtung insbesondere in dem Sinne unmittelbar vor dem zweiten Bildsensor angeordnet, dass zwischen der zweiten reflektierenden Fläche und dem zweiten Bildsensor keine weitere reflektierende Fläche angeordnet ist. Zwischen der zweiten reflektierenden Fläche und dem zweiten Bildsensor breitet Licht sich geradlinig oder im Wesentlichen geradlinig aus, und wird insbesondere allenfalls an Grenzflächen zwischen verschiedenen Materialien gebrochen.

In Lichtausbreitungsrichtung vor der ersten reflektierenden Fläche breitet Licht sich insbesondere entlang der optischen Achse des ersten Strahlengangs aus. Die optische Achse des ersten Strahlengangs ist vor der ersten reflektierenden Fläche insbesondere parallel oder im Wesentlichen parallel zu einer Längsachse des distalen Endbereichs des Schafts. In Lichtausbreitungsrichtung vor der zweiten reflektierenden Fläche breitet Licht sich insbesondere entlang der optischen Achse des zweiten Strahlengangs aus. Die optische Achse des zweiten Strahlengangs ist vor der zweiten reflektierenden Fläche insbesondere parallel oder im Wesentlichen parallel zu einer Längsachse des distalen Endbereichs des Schafts.

Bei einem Stereo-Endoskop, wie es hier beschrieben ist, sind der erste Bildsensor und der zweite Bildsensor insbesondere ferner in einer Richtung, die parallel zu zumindest entweder einer lichtempfindliche Schicht des ersten Bildsensors oder einer lichtempfindlichen Schicht des zweiten Bildsensors und orthogonal zu einer Längsachse des distalen Endbereichs des Schafts ist, relativ zu einander versetzt angeordnet, wobei der erste Bildsensor und der zweite Bildsensor in entgegengesetzte Richtungen orientiert sind.

Bei einem Stereo-Endoskop, wie es hier beschrieben ist, sind die erste Strahlengang-Ebene und die zweite Strahlengang-Ebene insbesondere parallel.

Bei einem Stereo-Endoskop, wie es hier beschrieben ist, ist ein Abstand zwischen der ersten Strahlengang-Ebene und der zweiten Strahlengang-Ebene insbesondere kleiner als die Länge einer Stereo-Basis des Stereo-Endoskops und ungleich Null.

Bei einem Stereo-Endoskop, wie es hier beschrieben ist, sind in einer Ebene orthogonal zu einer Längsachse des distalen Endbereichs des Schafts insbesondere der erste Bildsensor und der zweite Bildsensor an einander gegenüberliegenden Ecken eines Parallelogramms und die erste reflektierende Fläche und die zweite reflektierende Fläche an zwei weiteren einander gegenüberliegenden Ecken des Parallelogramms angeordnet. Die einander gegenüberliegenden Ecken sind nicht benachbarte Ecken.

Mit der Anordnung eines Bildsensors an einer Ecke eines Parallelogramms ist die Anordnung des Mittelpunkts des Bilderfassungsbereichs des Bildsensors an der Ecke des Parallelogramms gemeint. Mit der Anordnung einer reflektierenden Fläche an einer Ecke eines Parallelogramms ist gemeint, dass an der Ecke des Parallelogramms der Mittelpunkt der reflektierenden Fläche oder der Mittelpunkt des von Licht, das auf den Bilderfassungsbereich des Bildsensors gelenkt wird, erfüllten Bereichs der reflektierenden Fläche oder der Schnittpunkt der optischen Achse des Strahlengangs mit der reflektierenden Fläche angeordnet ist. Der Flächeninhalt des Parallelogramms ist größer als Null. Das Parallelogramm kann ein Rechteck und insbesondere ein Quadrat sein. Das Stereo-Endoskop enthält typischerweise keine Strukturen, die die Seiten des Parallelogramms nachzeichnen. Das Parallelogramm stellt also eine gedachte Form dar und ist nicht gegenständlich vorhanden. Die Seiten des Parallelogramms bilden an dessen Ecken, an denen die beiden reflektierenden Flächen, insbesondere die Mittelpunkte der reflektierenden Flächen, angeordnet sind, jeweils einen spitzen Winkel. An den Ecken, an denen die beiden Bildsensoren, insbesondere die Mittelpunkte der lichtempfindlichen Flächen der Bildsensoren, angeordnet sind, bilden die Seiten des Parallelogramms jeweils einen stumpfen Winkel.

Bei einigen herkömmlichen Stereo-Endoskopen sind entweder die Bildsensoren an benachbarten Ecken eines Rechtecks und die reflektierenden Flächen an benachbarten Ecken des Rechtecks oder die Bildsensoren und die reflektierenden Flächen auf einer einzigen Geraden angeordnet. Diese Anordnung ist jedoch nachteilig, da sie besonders viel Platz benötigt. Alle genannten Merkmale der Erfindung ermöglichen dagegen eine platzsparende Anordnung der Bildsensoren und reflektierenden Flächen im begrenzten Durchmesser des Endoskopschafts. Dadurch können Stereo-Instrumente mit besonders geringem Durchmesser geschaffen werden.

Bei einem Stereo-Endoskop, wie es hier beschrieben ist, sind die erste reflektierende Fläche und die zweite reflektierende Fläche insbesondere in zwei einander schneidenden Ebenen angeordnet.

Die beiden Ebenen, in denen die erste reflektierende Fläche und die zweite reflektierende Fläche angeordnet sind, sind insbesondere orthogonal oder im Wesentlichen orthogonal angeordnet. Die Schnittgerade der Ebene, in der die erste reflektierende Fläche angeordnet ist, und der Ebene, in der die zweite reflektierende Fläche angeordnet ist, schneidet insbesondere die Längsachse des distalen Endbereichs des Schafts.

Bei einem Stereo-Endoskop, wie es hier beschrieben ist, ist der zweite gerade Abschnitt des ersten Strahlengangs insbesondere neben dem zweiten Bildsensor angeordnet.

Bei einem Stereo-Endoskop, wie es hier beschrieben ist, ist der zweite gerade Abschnitt des zweiten Strahlengangs insbesondere neben dem ersten Bildsensor angeordnet.

Ein Bildsensor ist neben dem zweiten geraden Abschnitt eines Strahlengangs angeordnet, wenn eine Ebene, in der die lichtempfindliche Schicht des Bildsensors liegt, den zweiten geraden Abschnitt des Strahlengangs schneidet.

Bei einem Stereo-Endoskop, wie es hier beschrieben ist, ist eine lichtempfindliche Schicht des ersten Bildsensors insbesondere weder parallel noch orthogonal zu einer Basis-Ebene, in der der erste gerade Abschnitt des ersten Strahlengangs und der erste gerade Abschnitt des zweiten Strahlengangs liegen.

Bei einem Stereo-Endoskop, wie es hier beschrieben ist, ist eine lichtempfindliche Schicht des ersten Bildsensors insbesondere weder parallel noch orthogonal zu einer Stereo-Basis des Stereo-Endoskops.

Die Anordnung der lichtempfindlichen Schichten der Bildsensoren entweder parallel oder orthogonal zu einer Stereo-Basis ist eine Gemeinsamkeit vieler herkömmlicher Stereo-Endoskope. Bei der üblichen und für eine räumliche Wahrnehmung zumindest vorteilhaften horizontalen Wiedergabe der Stereo-Basis vereinfacht die Anordnung der lichtempfindlichen Schichten der Bildsensoren entweder parallel oder orthogonal zu der Stereo-Basis die Verarbeitung der Bilddaten. Insbesondere ist keine Rotation des erfassten Bilds um einen Winkel, der kein Vielfaches von 90 Grad ist, erforderlich.

Der Verzicht auf diese Einschränkung, also die Anordnung der lichtempfindlichen Schichten der Bildsensoren weder parallel noch orthogonal zu der Stereo-Basis erhöht den Aufwand, weil entweder die Bilddaten rotiert werden müssen oder der Bildschirm oder der Projektor zur Betrachtung des Stereobilds schräg angeordnet werden muss. Gleichzeitig können dadurch aber in dem distalen Endbereich des Schafts größere Bildsensoren angeordnet werden oder vorhandene Bildsensoren in einem kleineren Bauraum angeordnet werden. Damit kann das Verhältnis zwischen der Auflösung, also der Anzahl der Pixel oder Bildpunkte, einerseits und dem Schaftdurchmesser andererseits verbessert werden.

Bei einem Stereo-Endoskop, wie es hier beschrieben ist, liegt ein Winkel zwischen einer lichtempfindlichen Schicht des ersten Bildsensors und einer Stereo-Basis des Stereo-Endoskops insbesondere in einem Bereich von 10 Grad bis 80 Grad oder in einem Bereich von 20 Grad bis 70 Grad oder in einem Bereich von 30 Grad bis 60 Grad oder in einem Bereich von 40 Grad bis 50 Grad.

Bei einem Stereo-Endoskop, wie es hier beschrieben ist, weist der erste Bildsensor insbesondere einen ersten rechteckigen Bilderfassungsbereich auf, wobei ein Bild einer Geraden, die parallel zu einer Stereo-Basis des Stereo-Endoskops ist, in der lichtempfindlichen Schicht des ersten Bildsensors gegenüber geraden Randabschnitten des rechteckigen Bilderfassungsbereiches geneigt ist.

Diese Neigung erfordert die erwähnte Rotation.

Bei einem Stereo-Endoskop, wie es hier beschrieben ist, umfasst der distale Endbereich des Schafts insbesondere zwei gleiche oder im Wesentlichen gleiche Bauteile, die jeweils eine zylindrische Gestalt und einen im Wesentlichen halbkreisförmigen Querschnitt aufweisen.

Die beiden Bauteile können insbesondere im Fall eines Stereo-Endoskops, das nicht geradeaus blickt, sondern dessen Blickrichtung mit der Längsachse des distalen Endbereichs des Schafts einen Winkel, der größer als Null ist, einschließt, unterschiedlich orientierte Lichteintrittsflächen und unterschiedliche Strahlengänge unmittelbar hinter den Lichteintrittsflächen aufweisen. Insbesondere sind reflektierende Flächen unmittelbar hinter den Lichteintrittsflächen in beiden Bauteilen jeweils bezogen auf das Bauteil selbst entgegengesetzt geneigt.

Bei einem Stereo-Endoskop, wie es hier beschrieben ist, enthält insbesondere jedes der beiden Bauteile jeweils entweder den ersten Strahlengang oder den zweiten Strahlengang vollständig oder weitgehend.

Jedes der beiden Bauteile kann einen Strahlengang vollständig gekapselt enthalten. Alternativ kann jedes Bauteil eine seitliche Öffnung oder Ausnehmung aufweisen, die durch das jeweils andere Bauteil verschlossen wird.

Bei einem Stereo-Endoskop, wie es hier beschrieben ist, ist insbesondere eine Grenzfläche zwischen den beiden Bauteilen weder parallel noch orthogonal zu einer Stereo-Basis des Stereo-Endoskops.

Die Grenzfläche zwischen den beiden Bauteilen kann eben oder gekrümmt sein. Im Fall einer gekrümmten Grenzfläche ist insbesondere kein Teilbereich oder ein Teilbereich der gekrümmten Grenzfläche, der weniger als die Hälfte der Grenzfläche bildet, parallel oder orthogonal zu der Stereo-Basis.

Auf diese Weise können zwei zueinander komplementäre Bauteile geschaffen werden, die alle notwendigen Komponenten des Stereo-Endoskops enthalten und diese besonders platzsparend anordnen. Zusammen bilden die beiden Bauteile einen Teil des Schafts des Stereo-Endoskops mit möglichst geringem Durchmesser.

Bei einem Stereo-Endoskop, wie es hier beschrieben ist, weist eines der Bauteile insbesondere eine im Wesentlichen geschlossene Mantelfläche und eine Ausnehmung, die zu dem anderen Bauteil hin offen ist, und in der zumindest entweder der erste Bildsensor oder der zweite Bildsensor angeordnet ist, auf. Zusätzlich kann die Platine des ersten oder zweiten Bildsensors ganz oder teilweise in der Ausnehmung angeordnet sein.

Die geschlossene Mantelfläche weist insbesondere die Gestalt eines Ausschnitts einer Mantelfläche eines Zylinders mit kreisförmigem oder anderem Querschnitt auf. In der Ausnehmung sind insbesondere ferner die erste reflektierende Fläche und zumindest Teile des ersten Strahlengangs angeordnet. Die Ausnehmung weist insbesondere die Gestalt einer Tasche oder Nische, die zu der Grenzfläche zwischen den Bauteilen hin offen ist, auf. Durch Zusammenfügen der Bauteile werden die Ausnehmungen geschlossen, und es entsteht der distale Endbereich mit einer vollständig geschlossenen Mantelfläche.

Der distale Endbereich kann ferner ein oder mehrere weitere Bauteile umfassen, die beispielsweise die distale Stirnfläche des Schafts bilden.

Bei einem Stereo-Endoskop, wie es hier beschrieben ist, ragt der in dem ersten Bauteil angeordnete erste Bildsensor oder dessen Platine in eine Ausnehmung in dem zweiten Bauteil, und der in dem zweiten Bauteil angeordnete zweite Bildsensor oder dessen Platine ragt in eine Ausnehmung in dem ersten Bauteil.

So werden zusätzlich vorhandene Freiräume im jeweils gegenüberliegenden Bauteil genutzt, um im Querschnitt des Endoskops besonders platzraubende Komponenten des einen Bauteils, wie den Bildsensor und/oder dessen Platine, anzuordnen. Dadurch lassen sich der benötigte Bauraum und damit der Durchmesser des Instruments weiter verringern.

Ein Stereo-Endoskop, wie es hier beschrieben ist, umfasst insbesondere ein Prisma mit der ersten reflektierenden Fläche, wobei eine Kante des Prismas, die parallel zu der Längsachse des distalen Endbereichs des Schafts angeordnet ist, gefast oder abgeschrägt oder abgerundet ist.

Eine Fase oder eine Abschrägung oder eine Abrundung einer Kante - insbesondere außerhalb des von dem zur Bilderzeugung beitragenden Lichtbündel eingenommenen Raumbereichs - kann den von dem Prisma eingenommenen Bauraum verringern und eine kompaktere Bauweise ermöglichen. Die gefaste oder abgeschrägte oder abgerundete Kante des Prismas ist insbesondere nahe einer äußeren Oberfläche des distalen Endbereichs des Schafts angeordnet.

Bei einem Verfahren zum Erfassen eines mittels eines Stereo-Endoskops erzeugten Stereo-Bilds mit einem ersten Bild, das für die Betrachtung mit einem ersten Auge vorgesehen ist, und einem zweiten Bild, das für die Betrachtung mit einem zweiten Auge vorgesehen ist, wird ein erstes Lichtbündel, das von einem zu beobachtenden Objekt ausgeht, in dem Stereo-Endoskop in eine erste Richtung umgelenkt und fällt auf einen ersten Bildsensor, und wird ein zweites Lichtbündel, das von dem zu beobachtenden Objekt ausgeht, in dem Stereo-Endoskop in eine zweite Richtung umgelenkt und fällt auf einen zweiten Bildsensor, wobei die erste Richtung und die zweite Richtung entgegengesetzt oder im Wesentlichen entgegengesetzt sind.

Das Verfahren ist insbesondere mit einem Stereo-Endoskop ausführbar, wie es hier beschrieben ist. Ein Stereo-Endoskop, wie es hier beschrieben ist, ist insbesondere zur Ausführung eines Verfahrens, wie es hier beschrieben ist, vorgesehen und ausgebildet.

Bei einem Verfahren wie es hier beschrieben ist, sind sowohl die erste Richtung als auch die zweite Richtung insbesondere orthogonal oder im Wesentlichen orthogonal zu einer Längsachse eines distalen Endbereichs des Stereo-Endoskops.

Ein Verfahren, wie es hier beschrieben ist, umfasst insbesondere ferner einen Schritt des Verarbeitens von Bildsignalen, die von den Bildsensoren erzeugt sind, wobei bei dem Verarbeiten die durch die Bildsignale repräsentierten Bilder um einen vorbestimmten Winkel rotiert werden.

Der vorbestimmte Winkel liegt insbesondere in einem Bereich von 30 Grad bis 60 Grad, beispielsweise in einem Bereich von 40 Grad bis 50 Grad. Der vorbestimmte Winkel entspricht insbesondere dem Winkel zwischen Ebenen, in denen lichtempfindliche Bereiche der Bildsensoren liegen, und der Stereo-Basis des Stereo-Endoskops.

Auf diese Weise werden die auf dem Sensor rotiert aufgenommenen Bilder wieder an die Stereobasisebene angepasst.

Bei einem Verfahren zum Herstellen eines Stereo-Endoskops mit mehreren Strahlengängen werden mehrere gleiche Bauteile, die jeweils ein oder mehrere Bauteile in einem der mehreren Strahlengänge umfassen, gefertigt und anschließend in entgegengesetzten oder unterschiedlichen Orientierungen zusammengefügt, um zumindest einen Teil einer äußeren Oberfläche eines distalen Endbereichs des Stereo-Endoskops zu bilden.

Jedes der mehreren Bauteile umfasst insbesondere alle oder fast alle Bauteile von einem der mehreren Strahlengänge.

Im Falle zweier Bauteile werden diese insbesondere in entgegengesetzter Orientierung zusammengefügt. Im Falle dreier Bauteile werden diese insbesondere in Orientierungen, die sich jeweils paarweise um 120 Grad unterscheiden, zusammengefügt.

Die Bauteile können sich beispielsweise an ihren distalen Enden unterscheiden, um unterschiedliche Blickrichtungen zu ermöglichen.

### Kurzbeschreibung der Figuren

Nachfolgend werden Ausführungsformen anhand der beigefügten Figuren näher erläutert. Es zeigen:
- Figur 1: eine schematische Darstellung eines Stereo-Endoskops;
- Figur 2: eine schematische Darstellung eines distalen Endbereichs des Stereo-Endoskops aus Figur 1;
- Figur 3: eine weitere schematische Darstellung des distalen Endbereichs aus Figur 2;
- Figur 4: eine weitere schematische Darstellung des distalen Endbereichs aus den Figuren 2 und 3;
- Figur 5: eine weitere schematische Darstellung des distalen Endbereichs aus den Figuren 2 bis 4;
- Figur 6: eine weitere schematische Darstellung des distalen Endbereichs aus den Figuren 2 bis 5;
- Figur 7: eine schematische Darstellung eines distalen Endbereichs eines Schafts eines weiteren Stereo-Endoskops;
- Figur 8: eine weitere schematische Darstellung des distalen Endbereichs aus Figur 7;
- Figur 9: eine weitere schematische Darstellung des distalen Endbereichs aus den Figuren 7 und 8;
- Figur 10: eine schematische Darstellung eines distalen Endbereichs eines Schafts eines weiteren Stereo-Endoskops;
- Figur 11: eine weitere schematische Darstellung des distalen Endbereichs aus Figur 10.

### Beschreibung der Ausführungsformen

Figur 1 zeigt eine schematische Darstellung eines Stereo-Endoskops 10 mit einem Schaft 11. Der Schaft 11 weist ein distales Ende 12, einen das distale Ende 12 bildenden distalen Endbereich 13 und ein proximales Ende 14 auf. Zwischen dem proximalen Ende 14 und dem distalen Endbereich 13 ist der Schaft 11 flexibel, das heißt innerhalb konstruktiv gegebener Grenzen zerstörungsfrei elastisch oder plastisch verformbar. Der distale Endbereich 13 des Schafts 11 ist starr und gerade. Alternativ und abweichend von der Darstellung in Figur 1 kann der Schaft 11 starr und gekrümmt oder starr und gerade ausgebildet sein.

Figur 2 zeigt eine vergrößerte schematische Darstellung des distalen Endbereichs 13 des Schafts 11 des Stereo-Endoskops aus Figur 1. Der distale Endbereich 13 des Schafts 11 ist in Figur 2 transparent dargestellt, also nur durch Konturen angedeutet, so dass Einrichtungen innerhalb des Schafts 11 und des Endbereichs 13 sichtbar sind.

Das dargestellte Beispiel ist ein Stereo-Endoskop, das geradeaus blickt, dessen Blickrichtung also parallel zu der Längsachse 18 des distalen Endbereichs 13 des Schafts 11 ist. Die Längsachse 18 ist die Symmetrieachse, zu der die Mantelfläche des distalen Endstücks 13 translationssymmetrisch und/oder rotationssymmetrisch ist.

Der distale Endbereich 13 des Schafts 11 weist einen ersten Strahlengang 20 zu einem ersten Bildsensor 30 zum Erfassen eines ersten Bilds und einen zweiten Strahlengang 40 zu einem zweiten Bildsensor 50 zum Erfassen eines zweiten Bilds auf. Zur leichteren Unterscheidung sind in Figur 2 die optischen und elektronischen Bauelemente, die dem ersten Strahlengang zugeordnet sind, in dickeren und die optischen und elektronischen Bauelemente, die dem zweiten Strahlengang zugeordnet sind, in dünneren Linien dargestellt.

Das mittels des ersten Bildsensors 30 erfasste erste Bild ist insbesondere zur Betrachtung mit dem linken Auge vorgesehen, das mittels des zweiten Bildsensors 50 erfasste zweite Bild ist insbesondere zur Betrachtung mit dem rechten Auge vorgesehen. Die Strahlengänge 20, 30 sind in Figur 2 durch die optischen Achsen von in den Strahlengängen 20, 30 angeordneten optischen Bauteilen oder durch die geraden oder reflektierten Fortsetzungen dieser optischen Achsen angedeutet.

Der erste Strahlengang 20 umfasst innerhalb des distalen Endstücks 13 des Schafts 11 einen ersten geraden Abschnitt 23 und einen zweiten geraden Abschnitt 26. Der erste gerade Abschnitt 23 des ersten Strahlengangs 20 erstreckt sich von einer ersten Lichteintrittsfläche 21 bis zu einer ersten reflektierenden Fläche 25 in einem ersten Prisma 24. Der erste gerade Abschnitt 23 des ersten Strahlengangs 20 verläuft vor allem innerhalb eines ersten Objektivs 22, das in Figur 2 stark vereinfachend als zylindrisches Element angedeutet ist. Der zweite gerade Abschnitt 26 des ersten Strahlengangs 20 erstreckt sich von der ersten reflektierenden Fläche 25 bis zu einem dünnen schichtförmigen lichtempfindlichen Bereich 32 unter einer Lichteintrittsfläche 31 des ersten Bildsensors 30. Der erste gerade Abschnitt 23 des ersten Strahlengangs 20 ist parallel zu der Längsachse 18 des distalen Endbereichs 13 des Schafts 11. Beide gerade Abschnitte 23, 26 des ersten Strahlengangs 20 sind parallel zu der Zeichenebene der Figur 2.

Die erste reflektierende Fläche 25 ist in einem Winkel von ca. 45 Grad zu dem ersten Abschnitt 23 des ersten Strahlengangs 20 angeordnet. Deshalb beträgt der Winkel zwischen dem ersten Abschnitt 23 und dem zweiten Abschnitt 26 des ersten Strahlengangs 20 ca. 90 Grad. Der dünne schichtförmige lichtempfindliche Bereich 32 des ersten Bildsensors 30 ist orthogonal zu dem zweiten geraden Abschnitt 26 des ersten Strahlengangs 20 und parallel zu der Längsachse 18 des distalen Endbereichs 13 des Schafts 11.

Der zweite Strahlengang 40 umfasst innerhalb des distalen Endstücks 13 des Schafts 11 einen ersten geraden Abschnitt 43 und einen zweiten geraden Abschnitt 46. Der erste gerade Abschnitt 43 des zweiten Strahlengangs 40 erstreckt sich von einer zweiten Lichteintrittsfläche 41 bis zu einer zweiten reflektierenden Fläche 45 in einem zweiten Prisma 44. Der erste gerade Abschnitt 43 des zweiten Strahlengangs 40 verläuft vor allem innerhalb eines zweiten Objektivs 42, das in Figur 2 stark vereinfachend als zylindrisches Element angedeutet ist. Der zweite gerade Abschnitt 46 des zweiten Strahlengangs 40 erstreckt sich von der zweiten reflektierenden Fläche 45 bis zu einem dünnen schichtförmigen lichtempfindlichen Bereich unter einer Lichteintrittsfläche 51 des zweiten Bildsensors 50. Der erste gerade Abschnitt 43 des zweiten Strahlengangs 40 ist parallel zu der Längsachse 18 des distalen Endbereichs 13 des Schafts 11. Beide gerade Abschnitte 43, 46 des zweiten Strahlengangs 40 sind parallel zu der Zeichenebene der Figur 2.

Die zweite reflektierende Fläche 45 ist in einem Winkel von ca. 45 Grad zu dem ersten Abschnitt 43 des zweiten Strahlengangs 40 angeordnet. Deshalb beträgt der Winkel zwischen dem ersten Abschnitt 43 und dem zweiten Abschnitt 46 des zweiten Strahlengangs 40 ca. 90 Grad. Der dünne schichtförmige lichtempfindliche Bereich 52 des zweiten Bildsensors 50 ist orthogonal zu dem zweiten geraden Abschnitt 46 des zweiten Strahlengangs 40 und parallel zu der Längsachse 18 des distalen Endbereichs 13 des Schafts 11.

In der in Figur 2 dargestellten Perspektive liegen der erste Strahlengang 20 über oder vor und der zweite Strahlengang 40 unter oder hinter der Längsachse 18 des distalen Endbereichs 13 des Schafts 11.

Der erste Bildsensor 30 ist mit einer ersten Platine 36 mechanisch und elektrisch verbunden, der zweite Bildsensor 50 ist mit einer zweiten Platine 56 mechanisch und elektrisch verbunden. Die erste Platine 36 ist neben dem zweiten Prisma 44 angeordnet, liegt also in einer Ebene, die das zweite Prisma 44 schneidet. In der in Figur 2 dargestellten Perspektive liegt deshalb die erste Platine 36 vor oder über dem zweiten Prisma 44, verdeckt also das zweite Prisma 44 teilweise. Die zweite Platine 56 ist neben dem ersten Prisma 24 angeordnet, liegt also in einer Ebene, die das erste Prisma 24 schneidet. In der in Figur 2 dargestellten Perspektive liegt deshalb die zweite Platine 56 hinter oder unter dem ersten Prisma 24, wird also von dem ersten Prisma 24 teilweise verdeckt.

Die Ausbreitungsrichtung 27 von Licht, das von einem beobachteten Gegenstand ausgeht, in dem zweiten geraden Abschnitt 26 des ersten Strahlengangs 20 und die Ausbreitungsrichtung von Licht, das von einem beobachteten Gegenstand ausgeht, in dem in Figur 2 weitgehend hinter Einrichtungen des ersten Strahlengangs 20 verborgenen zweiten geraden Abschnitt 46 des zweiten Strahlengangs 40 sind entgegengesetzt. Entsprechend sind die Lichteintrittsflächen 31, 51 der Bildsensoren 30, 50 in entgegengesetzte Richtungen orientiert.

Figur 3 zeigt eine weitere schematische Darstellung des distalen Endbereichs 13 aus Figur 2. Die Art der Darstellung in Figur 3 entspricht weitgehend derjenigen der Figur 2, die Zeichenebene der Figur 3 ist ebenfalls parallel zu der Längsachse 18 des distalen Endbereichs 13 des Schafts 11, jedoch orthogonal zu der Zeichenebene der Figur 2.

Die Platinen 36, 56 sind parallel zu der Zeichenebene der Figur 3 angeordnet. Jede der beiden Platinen 36, 56 ist L-förmig. In der Perspektive der Figur 3 liegt die erste Platine 36 vor oder über der zweiten Platine 56 und verdeckt diese teilweise. In der Perspektive der Figur 3 ist ferner die erste Platine 36 vor oder über dem ersten Bildsensor 30 und dem ersten Prisma 25 angeordnet und verdeckt diese vollständig. Deshalb sind der erste Bildsensor 30 und das erste Prisma 25 eigentlich nicht sichtbar und in Figur 3 nur durch gestrichelte Linien angedeutet.

Die dünnen schichtförmigen lichtempfindlichen Bereiche 32, 52 der Bildsensoren 30, 50 sind innerhalb der Bildsensoren angeordnet und überdies durch die erste Platine 36 bzw. das zweite Prisma 44 verdeckt und deshalb eigentlich nicht sichtbar. Die Umrisse der dünnen schichtförmigen lichtempfindlichen Bereiche 32, 52 der Bildsensoren 30, 50 sind in Figur 3 trotzdem in durchgezogenen Linien angedeutet, um ihre rechteckige Gestalt mit geraden Randabschnitten 33, 34, 53, 54 zu zeigen. Die dünnen schichtförmigen lichtempfindlichen Bereiche 32, 52 der Bildsensoren 30, 50 sind parallel zu der Zeichenebene der Figur 3.

Der zweite gerade Abschnitt des ersten Strahlengangs 20 und der zweite gerade Abschnitt des zweiten Strahlengangs 40 sind orthogonal zu der Zeichenebene der Figur 3.

In Figur 3 sind ferner das Bild 38 einer Geraden, die parallel zu der Stereo-Basis des Stereo-Endoskops ist, in dem dünnen schichtförmigen lichtempfindlichen Bereich 32 des ersten Bildsensors 30 und das Bild 58 derselben Geraden in dem dünnen schichtförmigen lichtempfindlichen Bereich 52 des zweiten Bildsensors 50 dargestellt. Sowohl die erste reflektierende Schicht 25 und der dünne schichtförmige lichtempfindliche Bereich 32 des ersten Bildsensors 30 als auch die zweite reflektierende Schicht 45 und der dünne schichtförmige lichtempfindliche Bereich 52 des zweiten Bildsensors 50 sind schräg zu der Stereo-Basis des Stereo-Endoskops angeordnet. Deshalb sind sowohl das Bild 38 der Geraden in der dünnen schichtförmigen lichtempfindlichen Schicht 32 des ersten Bildsensors 30 als auch das Bild 58 der Geraden in der dünnen schichtförmigen lichtempfindlichen Schicht 52 des zweiten Bildsensors 30 schräg zu den dünnen schichtförmigen lichtempfindlichen Bereichen 32, 52 angeordnet und zu keinem von deren geraden Randabschnitten 33, 34, 53, 54 parallel.

Figur 4 zeigt eine schematische Darstellung eines Schnitts durch den distalen Endbereich 13 aus den Figuren 2 und 3. Die Schnittebene der Figur 4 ist orthogonal zu den Zeichenebenen der Figuren 2 und 3 und orthogonal zu der Längsachse 18 des distalen Endbereichs 13 des Schafts 11. Die Schnittebene der Figur 4 ist damit ferner orthogonal zu dem ersten geraden Abschnitt 23 des ersten Strahlengangs 20 und zu dem ersten geraden Abschnitt 43 des zweiten Strahlengangs 40.

Der distale Endbereich 13 des Schafts 11 umfasst zwei Bauteile 60, 70, die die mechanische Struktur und jeweils einen Teilbereich der äußeren Mantelfläche des distalen Endbereichs 13 bilden. Schnittflächen der Bauteile 60, 70 sind in Figur 4 schraffiert dargestellt.

Das erste Bauteil 60 weist eine äußere Mantelfläche 62, die einen Teil der äußeren Mantelfläche des distalen Endbereichs 13 des Schafts 11 bildet, auf. Ferner weist das erste Bauteil 60 eine Ausnehmung 64 auf. Die Ausnehmung 64 geht von einem dem zweiten Bauteil 70 zugewandten Oberflächenbereich 67 des ersten Bauteils 60 aus, öffnet sich also zu dem dem zweiten Bauteil 70 zugewandten Oberflächenbereich 67 hin. In der Ausnehmung 64 in dem ersten Bauteil 60 sind der erste Strahlengang 20, das erste Prisma 24 mit der ersten reflektierenden Fläche und der erste Bildsensor 30 teilweise oder - wie in dem Beispiel der Figur 4 - vollständig angeordnet.

Bei dem in Figur 4 dargestellten Beispiel ist eine Längskante (in Richtung parallel zu der Längsachse 18 des distalen Endbereichs 13 des Schafts 11 und damit orthogonal zu der Zeichenebene der Figur 4) des ersten Prismas 24 außerhalb des von dem Strahlenbündel eingenommenen Raums gefast oder abgeschrägt. Dies verringert den von dem ersten Prisma 24 eingenommenen Bauraum und ermöglicht eine kompaktere Bauweise.

Das zweite Bauteil 70 weist eine äußere Mantelfläche 72, die einen Teil der äußeren Mantelfläche des distalen Endbereichs 13 des Schafts 11 bildet, auf. Ferner weist das zweite Bauteil 70 eine Ausnehmung 74 auf. Die Ausnehmung 74 geht von einem dem ersten Bauteil 60 zugewandten Oberflächenbereich 76 des zweiten Bauteils 70 aus, öffnet sich also zu dem dem ersten Bauteil 60 zugewandten Oberflächenbereich 76 hin. In der Ausnehmung 74 in dem zweiten Bauteil 70 sind der zweite Strahlengang 40, das zweite Prisma 44 mit der ersten reflektierenden Fläche und der zweite Bildsensor 50 teilweise oder - wie in dem Beispiel der Figur 4 - vollständig angeordnet.

Bei dem in Figur 4 dargestellten Beispiel ist eine Längskante (in Richtung parallel zu der Längsachse 18 des distalen Endbereichs 13 des Schafts 11 und damit orthogonal zu der Zeichenebene der Figur 4) des zweiten Prismas 34 außerhalb des von dem Strahlenbündel eingenommenen Raums gefast oder abgeschrägt. Dies verringert den von dem zweiten Prisma 34 eingenommenen Bauraum und ermöglicht eine kompaktere Bauweise.

Bei der in Figur 4 angedeuteten Anordnung beider Bauteile 60, 70 bilden diese eine geschlossene Mantelfläche des distalen Endbereichs 13 des Schafts. Durch Fügen beider Bauteile 60, 70 - beispielsweise durch Laserschweißen - können beide Bauteile 60, 70 zu einer mechanischen Einheit verbunden werden, die die Strahlengänge 20, 40, die Prismen 24, 44 mit den reflektierenden Flächen 25, 45 und die Bildsensoren 30, 50 - zumindest in lateraler Richtung - hermetisch dicht umschließt. An einer distalen Stirnfläche kann der distale Endbereich 13 in jedem Bauteil 60, 70 ein hermetisch dicht eingesetztes Fensterbauteil, das eine der beiden Lichteintrittsflächen 21, 41 aufweist, aufweisen und somit ebenfalls hermetisch dicht verschlossen sein.

Bei dem in Figur 4 gezeigten Beispiel weist jedes der beiden Bauteile 60, 70 ferner einen Kanal 69, 79 in Form einer Bohrung parallel oder im Wesentlichen parallel zu der Längsachse 18 des distalen Endbereichs 13 des Schafts 11 auf. In den Kanälen 69, 79 können Lichtleitfasern angeordnet sein, die Beleuchtungslicht zu dem distalen Ende des Schafts 11 übertragen, das dort austritt und einen zu betrachteten Gegenstand beleuchtet. Alternativ oder zusätzlich kann in den Kanälen 69, 79 und/oder in einem oder mehreren weiteren Kanälen ein Fluid fließen und/oder ein Instrument zu dem distalen Ende des Stereo-Endoskops bewegt werden.

Figur 5 zeigt eine weitere schematische Darstellung des distalen Endbereichs 13 aus den Figuren 2 bis 4. Die Darstellung in Figur 5 entspricht weitgehend der Darstellung in Figur 4. In Figur 5 sind einige Bezugszeichen weggelassen und die Stereo-Basis 16 des Stereo-Endoskops angedeutet. Die Stereo-Basis ist die gerade Verbindungsstrecke der Schnittpunkte der optischen Achsen, die hier die Strahlengänge 20, 40 repräsentieren, mit den Lichteintrittsflächen 21, 41.

Ferner ist in Figur 5 ein Parallelogramm dargestellt, dessen Ecken durch die Mittelpunkte der reflektierenden Flächen (oder von deren an der Übertragung von Licht zu den Bildsensoren beteiligten Anteilen) und die Mittelpunkte der dünnen schichtförmigen lichtempfindlichen Bereiche 32, 52 der Bildsensoren 30, 50 angeordnet sind. Die reflektierenden Flächen und die Bildsensoren 30, 50 sind abwechselnd an den Ecken des Parallelogramms angeordnet. Die Mittelpunkte der reflektierenden Flächen sind an zwei einander gegenüberliegenden Ecken des Parallelogramms angeordnet. Die Mittelpunkte der lichtempfindlichen Bereiche 32, 52 der Bildsensoren 30, 50 sind an den anderen beiden einander gegenüberliegenden Ecken des Parallelogramms angeordnet. Die Seiten des Parallelogramms bilden an den Ecken, an denen die Mittelpunkte der reflektierenden Flächen angeordnet sind, jeweils einen spitzen Winkel. An den Ecken, an denen die Mittelpunkte der lichtempfindlichen Bereiche 32, 52 der Bildsensoren 30, 50 angeordnet sind, bilden die Seiten des Parallelogramms jeweils einen stumpfen Winkel.

Das in Figur 5 dargestellte Parallelogramm ist in dem distalen Endbereich 13 insbesondere nicht gegenständlich vorhanden. Der distale Endbereich weist also insbesondere keine Struktur auf, die beispielsweise eine der Seiten des Parallelogramms nachzeichnete. Lediglich die Mittelpunkte der reflektierenden Flächen und der Bildsensoren 30, 50 - die als solche aber auch nicht strukturell hervorgehoben sind - bilden die Ecken des gedachten Parallelogramms.

Figur 6 zeigt eine weitere schematische Darstellung des distalen Endbereichs 13 aus den Figuren 2 bis 5. Die Art der Darstellung in Figur 6 entspricht weitgehend derjenigen der Figuren 4 und 5.

Die Darstellung in Figur 6 unterscheidet sich von der Darstellung in den Figuren 4 und 5 insbesondere dadurch, dass die Bauteile 60, 70 mit den darin angeordneten Strahlengängen 20, 40, Prismen 24, 44, reflektierenden Flächen und Bildsensoren 30, 50 von einander beabstandet angeordnet sind. Die in Figur 6 dargestellte Situation liegt beispielsweise nach dem Einsetzen der Objektive, Prismen 24, 44, Bildsensoren mit Platinen 36, 56 in die Ausnehmungen 64, 74 der Bauteile 60, 70 und unmittelbar vor dem Zusammensetzen der beiden Bauteile 60, 70 vor.

Wie schon in den Figuren 3 bis 5 ist auch in Figur 6 erkennbar, dass das erste Prisma 24 mit der ersten reflektierenden Fläche 25, der erste Bildsensor 30 und damit der gesamte erste Strahlengang 20 in der Ausnehmung 64 in dem ersten Bauteil 60 angeordnet sind. Auch ein großer Teil der ersten Platine 36, mit der der erste Bildsensor 30 mechanisch und elektrisch verbunden ist, ist in der Ausnehmung 64 in dem ersten Bauteil 60 angeordnet. Ein Teil der ersten Platine 36 ragt jedoch aus der Ausnehmung 64 in dem ersten Bauteil 60 heraus. In der in den Figuren 2 bis 5 gezeigten zusammengesetzten Konfiguration oder Situation ist der aus der Ausnehmung 64 in dem ersten Bauteil 60 heraus ragende Teil der Platine 36 in der Ausnehmung 74 in dem zweiten Bauteil 70 aufgenommen.

Die beiden Bauteile 60, 70 einschließlich der darin angeordneten Prismen 24, 44 und Bildsensoren 30, 50 weisen insbesondere identische Abmessungen und mechanische, optische und elektrische Eigenschaften auf und sind lediglich im Raum entgegengesetzt orientiert angeordnet. Deshalb gilt für das zweite Bauteil 70 entsprechend, dass das zweite Prisma 44 mit der zweiten reflektierenden Fläche 45, der zweite Bildsensor 50 und damit der gesamte zweite Strahlengang 40 in der Ausnehmung 74 in dem zweiten Bauteil 70 angeordnet sind. Auch ein großer Teil der zweiten Platine 56, mit der der zweite Bildsensor 50 mechanisch und elektrisch verbunden ist, ist in der Ausnehmung 74 in dem zweiten Bauteil 70 angeordnet. Ein Teil der zweiten Platine 56 ragt jedoch aus der Ausnehmung 74 in dem zweiten Bauteil 70 heraus. In der in den Figuren 2 bis 5 gezeigten zusammengesetzten Konfiguration oder Situation ist der aus der Ausnehmung 74 in dem zweiten Bauteil 70 heraus ragende Teil der Platine 56 in der Ausnehmung 64 in dem ersten Bauteil 60 aufgenommen.

Bei dem anhand der Figuren 1 bis 6 dargestellten Beispiel sind der dem zweiten Bauteil 70 zugewandte Oberflächenbereich 67 des ersten Bauteils 60 und der dem ersten Bauteil 60 zugewandte Oberflächenbereich 76 des zweiten Bauteils 70 jeweils eben. Bei der in den Figuren 2 bis 5 gezeigten zusammengesetzten Konfiguration oder Situation liegen der ebene Oberflächenbereich 67 des ersten Bauteils 60 und der ebene Oberflächenbereich 76 des zweiten Bauteils 70 - von den Öffnungen zu den Ausnehmungen 64, 74 abgesehen - flächig an einander an. Zumindest die äußere Ränder oder Randbereiche der einander zugewandten und aneinander anliegenden Oberflächenbereiche 67, 76 sind gefügt, beispielsweise durch Laserschweißen.

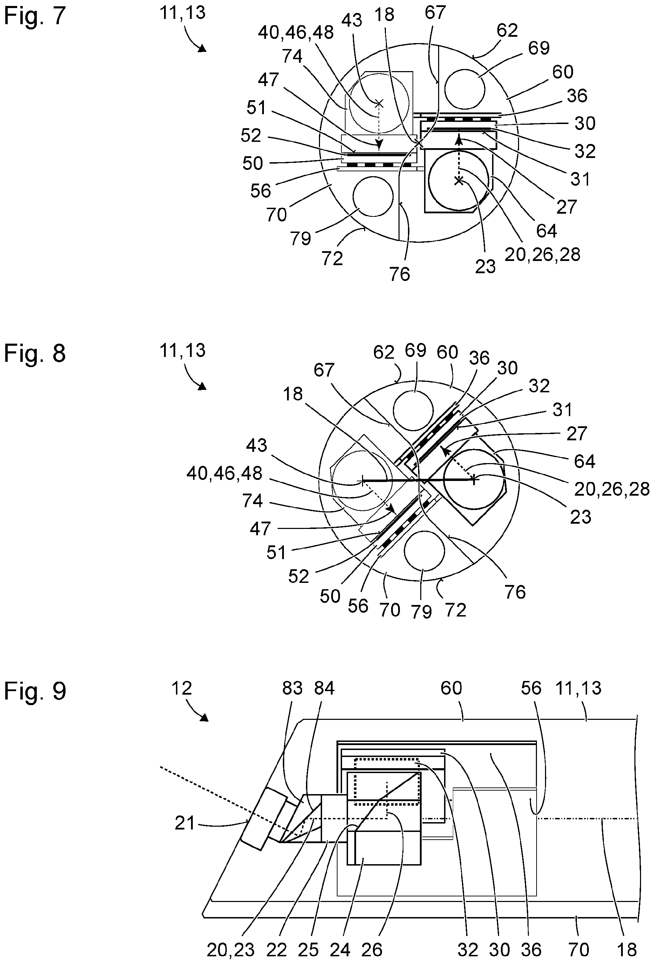

Figur 7 zeigt eine schematische Darstellung eines distalen Endbereichs 13 eines Schafts 11 eines weiteren Stereo-Endoskops, das dem anhand der Figuren 1 bis 6 dargestellten Stereo-Endoskop in einigen Merkmalen, Eigenschaften und Funktionen ähneln kann. Die Zeichenebene der Figur 7 ist parallel zu den Schnittebenen der Figuren 4, 5, 6 und orthogonal zu der Längsachse 18 des distalen Endbereichs 13 des Schafts 11. Anders als die Figuren 4, 5, 6 zeigt die Figur 7 keinen Schnitt, sondern eine frontale Draufsicht.

Nachfolgend sind insbesondere Merkmale, Eigenschaften und Funktionen beschrieben, in denen der in Figur 7 gezeigte distale Endbereich 13 des Schafts 11 sich von dem distalen Endbereich des Schafts des anhand der Figuren 1 bis 6 dargestellten Stereo-Endoskops unterscheidet.

Der in Figur 7 gezeigte distale Endbereich 13 des Schafts 11 eines Stereo-Endoskops 10 unterscheidet sich von dem distalen Endbereich des Schafts des anhand der Figuren 1 bis 6 dargestellten Stereo-Endoskops insbesondere dadurch, dass die Grenzfläche zwischen den Bauteilen 60, 70 nicht eben, sondern gekrümmt ist. Der dem zweiten Bauteil 70 zugewandte Oberflächenbereich 67 des ersten Bauteils 60 und der dem ersten Bauteil 60 zugewandte Oberflächenbereich 76 des zweiten Bauteils 70 sind - von den Öffnungen zu den Ausnehmungen 64, 74 abgesehen - einander entsprechend gekrümmt. Deshalb liegen auch bei dem in Figur 7 gezeigten Beispiel der Oberflächenbereich 67 des ersten Bauteils 60 und der Oberflächenbereich 76 des zweiten Bauteils 70 - von den Öffnungen zu den Ausnehmungen 64, 74 abgesehen - flächig aneinander an. Zumindest die äußere Ränder oder Randbereiche der einander zugewandten Oberflächenbereiche 67, 76 sind gefügt, beispielsweise durch Laserschweißen.

Bei dem in Figur 7 gezeigten Beispiel sind die gekrümmten Oberflächenbereiche 67, 76 der Bauteile jeweils zylindersymmetrische, also translationsinvariant hinsichtlich einer Translation parallel zu der Längsachse 18 des distalen Endbereichs 13 des Schafts 11.

Die Krümmung der einander zugewandten und - von den Öffnungen zu den Ausnehmungen 64, 74 abgesehen - aneinander anliegenden Oberflächenbereiche 67, 76 kann die Fertigung, insbesondere die Erzeugung der Ausnehmungen 64, 74, vereinfachen, und beispielsweise eine Vergrößerung der Ausnehmungen 64, 74 gegenüber dem Fall ebener Oberflächenbereiche vereinfachen.

Figur 8 zeigt eine weitere schematische Darstellung des distalen Endbereichs 13 des Schafts 11 aus Figur 7. Die Art der Darstellung in Figur 8 entspricht weitgehend derjenigen der Figur 7. Insbesondere entspricht die Zeichenebene der Figur 8 der Zeichenebene der Figur 7.

Die Darstellung in Figur 8 unterscheidet sich von der Darstellung in Figur 7 dadurch, dass der distale Endbereich 13 des Schafts 11 um seine Längsachse 18 rotiert ist, so dass die Stereo-Basis 16 des Stereo-Endoskops in Figur 8 horizontal angeordnet ist. Ferner sind in Figur 8 einige Bezugszeichen weggelassen.

Figur 9 zeigt eine weitere schematische Darstellung des distalen Endbereichs 13 des Schafts 11 aus den Figuren 7 und 8. Die Art der Darstellung in Figur 9 ähnelt derjenigen der Figuren 2 und 3. Insbesondere ist der distale Endbereich 13 des Schafts 11 in Figur 9 transparent dargestellt, also nur durch Konturen und den Rand der Grenzfläche zwischen den Bauteilen 60, 70 angedeutet, so dass Einrichtungen innerhalb des Endbereichs 13 des Schafts 11 sichtbar sind. Die Zeichenebene der Figur 9 ist parallel zu der Längsachse 18 des distalen Endbereichs 13 des Schafts 11, orthogonal zu der Zeichenebene der Figur 8 und - anders als die Zeichenebenen der Figuren 2, 3 - orthogonal zu der Stereo-Basis des Stereo-Endoskops. Die beiden Strahlengänge 20, 40 mit den Prismen 24, 44, den reflektierenden Flächen 25, 45 und den Bildsensoren 30, 50 (vgl. Figuren 7, 8) liegen bei dieser Darstellung in Figur 9 exakt hintereinander. Der erste Strahlengang 30 verdeckt also den zweiten Strahlengang. Von der ersten reflektierenden Fläche 25 ist in Figur 9 der Rand an der äußeren Oberfläche des Prismas 24 sichtbar.

Aus Gründen der Übersichtlichkeit ist in Figur 9 ferner lediglich die zweite Platine 56, nicht aber der durch das erste Prisma 24 und die zweite Platine 56 nur weitgehend, aber tatsächlich nicht vollständig verdeckte zweite Bildsensor dargestellt.

Das erste Prisma 24, der erste Bildsensor 30 und die Platinen 36, 56 sind bei der Perspektive der Figur 9 aus einer Richtung sichtbar, die weder parallel noch orthogonal zu den ebenen äußeren Oberflächenbereichen dieser Komponenten ist (vgl. Figur 8).

In Figur 9 ist erkennbar, dass die Blickrichtung des in den Figuren 7 bis 9 gezeigten distalen Endbereichs 13 des Schafts 11 anders als bei dem anhand der Figuren 2 bis 6 dargestellten distalen Endbereich 13 nicht parallel zu der Längsachse 18 des distalen Endbereichs 13 des Schafts 11 ist. Dazu ist in dem ersten Strahlengang 20 ein Blickrichtungsprisma 83 mit mehreren reflektierenden Flächen 84 vorgesehen. Das Blickrichtungsprisma 83 ist distal des ersten Prismas 24 so angeordnet, dass alle reflektierenden Flächen 84 des Blickrichtungsprimas 83 orthogonal zu der Zeichenebene der Figur 9 sind. Deshalb liegt der gesamte erste Strahlengang distal - das heißt in Lichtausbreitungsrichtung vor - der ersten reflektierenden Fläche 25 des ersten Prismas 24, in einer Ebene parallel zu der Zeichenebene der Figur 9. Der zweite gerade Abschnitt 26 des ersten Strahlengangs 20, der sich in Lichtausbreitungsrichtung hinter der ersten reflektierenden Fläche 25 des ersten Prismas 24 befindet, ist nicht parallel zu der Zeichenebene der Figur 9.

Entsprechendes gilt für den identischen, jedoch in Figur 9 vollständig durch den ersten Strahlengang 20 verdeckten zweiten Strahlengang.

Bei dem in den Figuren 7 bis 9 gezeigten Beispiel bilden die Bauteile 60, 70 zusammen die gesamte äußere Oberfläche des distalen Endbereichs 13 des Schafts 11, also auch dessen distale Stirnfläche.

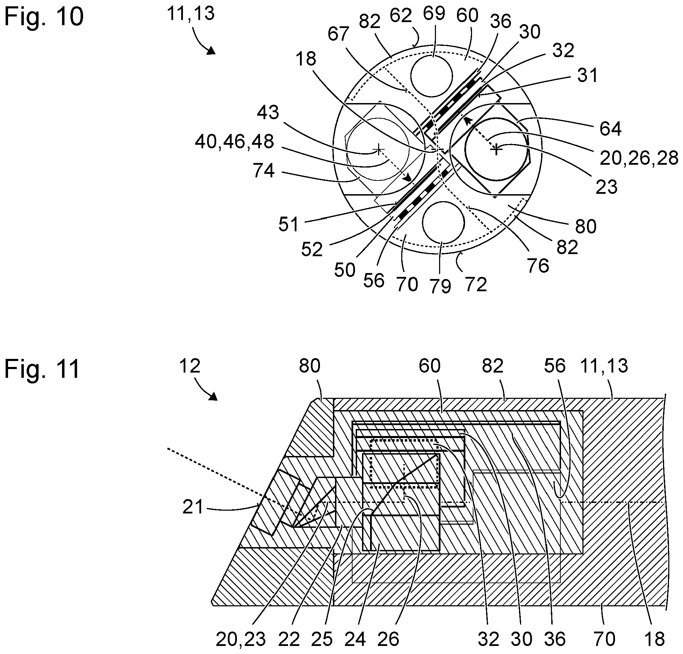

Figur 10 zeigt eine schematische Darstellung eines distalen Endbereichs 13 eines Schafts 11 eines weiteren Stereo-Endoskops, das dem anhand der Figuren 1 bis 6 dargestellten Stereo-Endoskop und vor allem dem anhand der Figuren 7 bis 9 dargestellten Stereo-Endoskop in einigen Merkmalen, Eigenschaften und Funktionen ähnelt. Die Art der Darstellung in Figur 10 entspricht derjenigen der Figuren 7 und vor allem 8. Insbesondere ist die Zeichenebene der Figur 10 orthogonal zu der Längsachse 18 des distalen Endbereichs 13 des Schafts 11.

Nachfolgend sind insbesondere Merkmale, Eigenschaften und Funktionen beschrieben, in denen der in Figur 10 gezeigte distale Endbereich 13 des Schafts 11 sich von dem distalen Endbereich des Schafts des anhand der Figuren 7 bis 9 dargestellten Stereo-Endoskop unterscheidet.

Der in Figur 10 gezeigte distale Endbereich 13 des Schafts 11 eines Stereo-Endoskops 10 unterscheidet sich von dem distalen Endbereich des Schafts des anhand der Figuren 7 bis 9 dargestellten Stereo-Endoskops insbesondere dadurch, dass die Bauteile 60, 70 nicht die gesamte äußere Oberfläche des distalen Endbereichs 13 des Schafts 11 bilden. Stattdessen bilden die Bauteile 60, 70 nur einen Teil der Mantelfläche des distalen Endbereiches 13 des Schafts 11 und nur Teile von dessen distaler Stirnfläche, die bei der Darstellung in Figur 10 dem Betrachter zugewandt ist. Weitere Teile der äußeren Oberfläche des distalen Endbereichs 13 des Schafts 11 werden durch ein drittes Bauteil 80 und durch ein viertes Bauteil 82 gebildet. Das dritte Bauteil 80 weist in der Betrachtungsrichtung der Figur 10 näherungsweise die Gestalt eines liegenden und mehrfach stark abgerundeten Großbuchstabens ,H' auf und bildet einen entsprechenden Teil der in Figur 10 dem Betrachter zugewandten Frontfläche des distalen Endbereichs 13 des Schafts 11 des Stereo-Endoskops. Das vierte Bauteil 82 bildet einen Teil der Mantelfläche des Stereo-Endoskops 10. Die Konturen dünnwandiger und zungenförmig nach distal reichender Bereiche des vierten Bauteils 82 sind in Figur 10 durch gestrichelte Linien angedeutet.

Figur 11 zeigt eine weitere schematische Darstellung des distalen Endbereichs 13 des Schafts 11 aus Figur 10. Die Art der Darstellung in Figur 11 ähnelt derjenigen der Figuren 2, 3 und vor allem 9. Insbesondere ist der distale Endbereich 13 des Schafts 11 in Figur 11 transparent dargestellt, also durch Konturen angedeutet, so dass Einrichtungen innerhalb des Endbereichs 13 des Schafts 11 sichtbar sind. Die Zeichenebene der Figur 11 ist parallel zu der Längsachse 18 des distalen Endbereichs 13 des Schafts 11, orthogonal zu der Zeichenebene der Figur 10 und - wie die Zeichenebene der Figur 9 - orthogonal zu der Stereo-Basis des Stereo-Endoskops. Die beiden in Figur 10 sichtbaren Strahlengänge 20, 40 mit den Prismen 24, 44, den reflektierenden Flächen 25, 45 und den Bildsensoren 30, 50 liegen bei dieser Darstellung in Figur 11 exakt hintereinander. Der erste Strahlengang 20 verdeckt also den zweiten Strahlengang.

Aus Gründen der Übersichtlichkeit ist in Figur 11 ferner lediglich die zweite Platine 56, nicht aber der durch das erste Prisma 24 und die zweite Platine 56 nur weitgehend, aber tatsächlich nicht vollständig verdeckte zweite Bildsensor dargestellt.

Das erste Bauteil 60 und das in Figur 11 nicht sichtbare zweite Bauteil bilden nur einen Teil der äußeren Oberfläche des distalen Endbereichs 13 des Schafts 11. Zur Unterscheidung von anderen Bauteilen, die Teile der äußeren Oberfläche des distalen Endbereichs 13 bilden, ist derjenige Teile der äußeren Oberfläche, der von dem ersten Bauteil 60 gebildet ist, steil und weit von links oben nach rechts unten schraffiert.

Das dritte Bauteil 80 bildet einen großen Teil der distalen Stirnfläche und zwei Teilbereiche (in Figur 11: oben und unten) eines sich daran anschließenden ringförmigen Bereichs der Mantelfläche. Zur Unterscheidung von demjenigen Teil der äußeren Oberfläche, der durch das erste Bauteil 60 gebildet ist, ist derjenige Teil der äußeren Oberfläche, der durch das dritte Bauteil 80 gebildet ist, enger und flacher (von links oben nach rechts unten) schraffiert. In der Zusammenschau der Figuren 10 und 11 ist erkennbar, dass die Gestalt des dritten Bauteils 80 näherungsweise ein kreisförmiger Ausschnitt aus einem Keil ist. Zwei breite und tiefe Nuten parallel zu der Längsachse 18 des distalen Endbereichs 13, die von einander gegenüberliegenden Randbereichen ausgehen, weisen bei dem in den Figuren 10, 11 dargestellten Beispiel jeweils einen hufeisenförmigen Querschnitt auf. Diese Nuten sind durch entsprechende Bereiche des ersten Bauteils 60 und des zweiten Bauteils 70 mit korrespondierender Gestalt, die die Strahlengänge 20, 30 umgeben, vollständig ausgefüllt.

Zumindest die Ränder oder Randbereiche der Grenzflächen zwischen dem dritten Bauteil 80 und dem ersten Bauteil 60 und dem zweiten Bauteil 70 sind gefügt, beispielsweise durch Laserschweißen.

Ein rohrförmiges viertes Bauteil 82 schließt sich nach proximal an das erste Bauteil 60 und das zweite Bauteil 70 an. Zur Unterscheidung von Teilen der äußeren Oberfläche des distalen Endbereichs 13 des Schafts 11, die durch das erste Bauteil 60 oder durch das dritte Bauteil 80 gebildet sind, ist das rohrförmige vierte Bauteil 82 in Figur 11 enger und von links unten nach rechts oben schraffiert. Das distale Ende des vierten Bauteils 82 weist zwei Zungen auf, die zwischen den durch das erste Bauteil 60 und das in Figur 11 nicht sichtbare zweite Bauteil gebildeten Teilen der äußeren Oberfläche die äußere Oberfläche des distalen Endbereichs 13 bilden und an das dritte Bauteil 80 angrenzen.

Abweichend von dem anhand der Figuren 1 bis 6 dargestellten Beispiel können auch bei einem Stereo-Endoskop 10 mit gerader Blickrichtung, also mit Blickrichtung parallel zu der Längsachse des distalen Endbereichs 13 des Schafts 11, die an einander anliegenden und miteinander gefügten Oberflächenbereiche 67, 76 der Bauteile 60, 70 gekrümmt sein. Abweichend von den anhand der Figuren 7 bis 11 dargestellten Beispielen können auch bei einem Stereo-Endoskop 10 mit gerader Blickrichtung, also mit Blickrichtung parallel zu der Längsachse des distalen Endbereichs 13 des Schafts 11, die an einander anliegenden und miteinander gefügten Oberflächenbereiche 67, 76 der Bauteile 60, 70 eben sein.

Abweichend von dem anhand der Figuren 1 bis 6 dargestellten Beispiel kann auch bei einem Stereo-Endoskop 10 mit Blickrichtung parallel zur Längsachse 18 des distalen Endbereichs 13 des Schafts 11 die äußere Oberfläche des distalen Endbereichs 13 teilweise durch ein drittes Bauteils 80 oder durch ein drittes Bauteil 80 und ein viertes Bauteil 82 oder durch mehrere weitere Bauteile gebildet sein.

### Bezugszeichen

- **10**: **Stereo-Endoskop**
- 11: Schaft des Stereo-Endoskops 10
- 12: distales Ende des Schafts 11
- 13: distaler Endbereich des Schafts 11
- 14: proximales Ende des Schafts 11
- 16: Stereo-Basis des Stereo-Endoskops 10
- 18: Längsachse des distalen Endbereichs 13 des Schafts
- **20**: **erster Strahlengang des Stereo-Endoskops 10**
- 21: Lichteintrittsfläche des ersten Strahlengangs 20
- 22: Objektiv in dem ersten Strahlengang 20
- 23: erster gerader Abschnitt des ersten Strahlengangs 20
- 24: erstes Prisma in dem ersten Strahlengang 20
- 25: erste reflektierende Fläche in dem ersten Prisma 24
- 26: zweiter gerader Abschnitt des ersten Strahlengangs 20
- 27: Lichtausbreitungsrichtung in dem ersten Strahlengang 20
- 28: erste Strahlengang-Ebene, in der der erste Strahlengang 20 liegt
- **30**: **erster Bildsensor des Stereo-Endoskops 10**
- 31: Lichteintrittsfläche des ersten Bildsensors 30
- 32: lichtempfindliche Schicht, lichtempfindlicher Bereich des ersten Bildsensors 30
- 33: gerader Randabschnitt des rechteckigen lichtempfindlichen Bereichs 32
- 34: gerader Randabschnitt des rechteckigen lichtempfindlichen Bereichs 32
- 36: erste Platine, an der der erste Bildsensor 30 angeordnet ist
- 38: Bild einer Geraden parallel zu der Stereo-Basis 16 des Stereo-Endoskops 10 in der lichtempfindlichen Schicht 32 des ersten Bildsensors 30
- **40**: **zweiter Strahlengang des Stereo-Endoskops 10**
- 41: Lichteintrittsfläche des zweiten Strahlengangs 40
- 42: Objektiv in dem zweiten Strahlengang 40
- 43: erster gerader Abschnitt des zweiten Strahlengangs 40
- 44: zweites Prisma in dem zweiten Strahlengang 40
- 45: zweite reflektierende Fläche in dem zweiten Prisma 44
- 46: zweiter gerader Abschnitt des zweiten Strahlengangs 40
- 47: Lichtausbreitungsrichtung in dem zweiten Strahlengang 40
- 48: zweite Strahlengang-Ebene, in der der zweite Strahlengang 40 liegt
- **50**: **zweiter Bildsensor des Stereo-Endoskops 10**
- 51: Lichteintrittsfläche des zweiten Bildsensors 50
- 52: lichtempfindliche Schicht, lichtempfindlicher Bereich des ersten Bildsensors 50
- 53: gerader Randabschnitt des rechteckigen lichtempfindlichen Bereichs 52
- 54: gerader Randabschnitt des rechteckigen lichtempfindlichen Bereichs 52
- 56: zweite Platine, an der der zweite Bildsensor 50 angeordnet ist
- 58: erstes Bild einer Geraden parallel zu der Stereo-Basis 16 des Stereo-Endoskops 10 in der lichtempfindlichen Schicht 52 des zweiten Bildsensors 50
- **60**: **erstes Bauteil des distalen Endbereichs 13 des Schafts 11**
- 62: Mantelfläche des ersten Bauteils 60
- 64: Ausnehmung in dem ersten Bauteil 60, in der die erste reflektierende Fläche 24 und der erste Bildsensor 30 angeordnet sind
- 67: dem zweiten Bauteil 70 zugewandter Oberflächenbereich des ersten Bauteils 60
- 69: Kanal für Lichtleitfasern für Beleuchtungslicht in dem ersten Bauteil 60
- **70**: **zweites Bauteil des distalen Endbereichs 13 des Schafts 11**
- 72: Mantelfläche des zweiten Bauteils 70
- 74: Ausnehmung in dem zweiten Bauteil 70, in der die zweite reflektierende Fläche 44 und der zweite Bildsensor 50 angeordnet sind
- 76: dem ersten Bauteil 60 zugewandter Oberflächenbereich des zweiten Bauteils 70
- 79: Kanal für Lichtleitfasern für Beleuchtungslicht in dem zweiten Bauteil 70
- 80: drittes Bauteil des distalen Endbereichs 13 des Schafts 11
- 82: rohrförmiges viertes Bauteil des distalen Endbereichs 13 des Schafts 11
- 83: Blickrichtungsprisma in dem ersten Strahlengang
- 84: reflektierende Fläche des Blickrichtungsprismas in dem ersten Strahlengang
- 86: erste Richtung, in der die Bildsensoren relativ zu einander versetzt angeordnet sind
- 87: zweite Richtung, in der die Bildsensoren relativ zu einander versetzt angeordnet sind

## Patentansprüche

1. Stereo-Endoskop (10) zum Erfassen eines für die Betrachtung mit einem ersten Auge vorgesehenen ersten Bilds und eines für die Betrachtung mit einem zweiten Auge vorgesehenen zweiten Bilds, mit:
einem Schaft (11) mit einem distalen Endbereich (13);
einem ersten Bildsensor (30) in dem distalen Endbereich (13) des Schafts (11), zum Erfassen des ersten Bilds;
einem zweiten Bildsensor (50) in dem distalen Endbereich (13) des Schafts (11), zum Erfassen des zweiten Bilds,
wobei sowohl die lichtempfindliche Schicht (32) des ersten Bildsensors (30) als auch die lichtempfindliche Schicht (52) des zweiten Bildsensors (50) parallel oder im Wesentlichen parallel zu einer Längsachse (18) des distalen Endbereichs (13) angeordnet ist,
wobei der erste Bildsensor (30) und der zweite Bildsensor (50) in entgegengesetzte oder im Wesentlichen entgegengesetzte Richtungen orientiert sind,
wobei der erste Bildsensor (30) und der zweite Bildsensor (50) in einer Richtung (86), die orthogonal zu einer Längsachse (18) des distalen Endbereichs (13) des Schafts (11) und parallel zu zumindest entweder einer lichtempfindlichen Schicht (32) des ersten Bildsensors (30) oder einer lichtempfindlichen Schicht (52) des zweiten Bildsensors (50) ist, relativ zu einander versetzt angeordnet sind,
**dadurch gekennzeichnet, dass** der erste Bildsensor (30) einen ersten rechteckigen Bilderfassungsbereich (32) aufweist,
und ein Bild (38) einer Geraden, die parallel zu einer Stereo-Basis (16) des Stereo-Endoskops (10) ist, in der lichtempfindlichen Schicht (32) des ersten Bildsensors (30) gegenüber geraden Randabschnitten (33, 34) des rechteckigen Bilderfassungsbereiches (32) geneigt ist.

2. Stereo-Endoskop (10) gemäß dem vorangehenden Anspruch, bei dem eine lichtempfindliche Schicht (32) des ersten Bildsensors (30) und eine lichtempfindliche Schicht (52) des zweiten Bildsensors (50) in einer Richtung (87), die orthogonal zu den lichtempfindlichen Schichten (32, 52) beider Bildsensoren (30, 50) und orthogonal zu einer Längsachse (18) des distalen Endbereichs (13) des Schafts (11) ist, relativ zueinander versetzt angeordnet sind.

3. Stereo-Endoskop (10) gemäß einem der vorangehenden Ansprüche, bei dem der distale Endbereich (13) des Schafts (11) zwei gleiche oder im Wesentlichen gleiche Bauteile (60, 70), die jeweils eine zylindrische Gestalt und einen im Wesentlichen halbkreisförmigen Querschnitt aufweisen, umfasst.

4. Stereo-Endoskop (10) gemäß Anspruch 3, bei dem eine Grenzfläche (67, 76) zwischen den beiden Bauteilen (60, 70) weder parallel noch orthogonal zu einer Stereo-Basis (16) des Stereo-Endoskops (10) ist.

5. Stereo-Endoskop (10) gemäß einem der Ansprüche 3 oder 4, bei dem eines der Bauteile (60) aufweist
eine im Wesentlichen geschlossene Mantelfläche (62),
eine Ausnehmung (64), die zu dem anderen Bauteil (70) hin offen ist, und in der zumindest entweder der erste Bildsensor (30) oder der zweite Bildsensor (50) angeordnet ist.

6. Stereo-Endoskop (10) gemäß einem der Ansprüche 3 bis 5, bei dem der in dem ersten Bauteil (60) angeordnete erste Bildsensor (30) oder dessen Platine (36) in eine Ausnehmung (74) in dem zweiten Bauteil (70) ragt und der in dem zweiten Bauteil (70) angeordnete zweite Bildsensor (50) oder dessen Platine (56) in eine Ausnehmung (64) in dem ersten Bauteil (60) ragt.

7. Stereo-Endoskop (10) zum Erfassen eines für die Betrachtung mit einem ersten Auge vorgesehenen ersten Bilds und eines für die Betrachtung mit einem zweiten Auge vorgesehenen zweiten Bilds, mit:
einem Schaft (11) mit einem distalen Endbereich (13);
einem ersten Bildsensor (30) in dem distalen Endbereich (13) des Schafts (11), zum Erfassen des ersten Bilds;
einem zweiten Bildsensor (50) in dem distalen Endbereich (13) des Schafts (11), zum Erfassen des zweiten Bilds;
einer ersten reflektierenden Fläche (25) zum Reflektieren von Licht;
einer zweiten reflektierenden Fläche (45) zum Reflektieren von Licht;
einem ersten Strahlengang (20) mit einem ersten geraden Abschnitt (23), der sich in Lichtausbreitungsrichtung bis zu der ersten reflektierende Fläche (25) erstreckt, und einem zweiten geraden Abschnitt (26), der sich von der ersten reflektierenden Fläche (25) bis zu dem ersten Bildsensor (30) erstreckt;
einem zweiten Strahlengang (40) mit einem ersten geraden Abschnitt (43), der sich in Lichtausbreitungsrichtung bis zu der zweiten reflektierende Fläche (45) erstreckt, und einem zweiten geraden Abschnitt (46), der sich von der zweiten reflektierenden Fläche (45) bis zu dem zweiten Bildsensor (50) erstreckt;
wobei der erste gerade Abschnitt (23) und der zweite gerade Abschnitt (26) des ersten Strahlengangs (20) in einer ersten Strahlengang-Ebene (28) angeordnet sind,
wobei der erste gerade Abschnitt (43) und der zweite gerade Abschnitt (46) des zweiten Strahlengangs (40) in einer zweiten Strahlengang-Ebene (48), die von der ersten Strahlengang-Ebene (28) verschieden ist, angeordnet ist,
wobei eine Lichtausbreitungsrichtung (27) von Licht in dem zweiten geraden Abschnitt (26) des ersten Strahlengangs (20) und eine Lichtausbreitungsrichtung (47) von Licht in dem zweiten geraden Abschnitt (46) des zweiten Strahlengangs (40) entgegengesetzt oder im Wesentlichen entgegengesetzt sind,
**dadurch gekennzeichnet, dass** eine lichtempfindliche Schicht (32) des ersten Bildsensors (30) weder parallel noch orthogonal ist zu einer Basis-Ebene, in der der erste gerade Abschnitt (23) des ersten Strahlengangs (20) und der erste gerade Abschnitt (43) des zweiten Strahlengangs (40) liegen.

8. Stereo-Endoskop (10) gemäß Anspruch 7, bei dem der zweite gerade Abschnitt (26) des ersten Strahlengangs (20) neben dem zweiten Bildsensor (50) angeordnet ist.

9. Stereo-Endoskop (10) gemäß einem der Ansprüche 7 oder 8, bei dem der erste Bildsensor (30) einen ersten rechteckigen Bilderfassungsbereich (32) aufweist, ein Bild (38) einer Geraden, die parallel zu einer Stereo-Basis (16) des Stereo-Endoskops (10) ist, in der lichtempfindlichen Schicht (32) des ersten Bildsensors (30) gegenüber geraden Randabschnitten (33, 34) des rechteckigen Bilderfassungsbereiches (32) geneigt ist.

10. Stereo-Endoskop (10) gemäß einem der Ansprüche 7 bis 9, bei dem der distale Endbereich (13) des Schafts (11) zwei gleiche oder im Wesentlichen gleiche Bauteile (60, 70), die jeweils eine zylindrische Gestalt und einen im Wesentlichen halbkreisförmigen Querschnitt aufweisen, umfasst.

11. Stereo-Endoskop (10) gemäß dem vorangehenden Anspruch, bei dem jedes der beiden Bauteile (60, 70) jeweils entweder den ersten Strahlengang (20) oder den zweiten Strahlengang (40) vollständig oder weitgehend enthält.

12. Stereo-Endoskop (10) gemäß einem der Ansprüche 10 und 11, bei dem eine Grenzfläche (67, 76) zwischen den beiden Bauteilen (60, 70) weder parallel noch orthogonal zu einer Stereo-Basis (16) des Stereo-Endoskops (10) ist.

13. Stereo-Endoskop (10) gemäß einem der Ansprüche 10 bis 12, bei dem eines der Bauteile (60) aufweist
eine im Wesentlichen geschlossene Mantelfläche (62),
eine Ausnehmung (64), die zu dem anderen Bauteil (70) hin offen ist, und in der zumindest entweder der erste Bildsensor (30) oder der zweite Bildsensor (50) angeordnet ist.

14. Stereo-Endoskop (10) gemäß einem der Ansprüche 10 bis 13, bei dem der in dem ersten Bauteil (60) angeordnete erste Bildsensor (30) oder dessen Platine (36) in eine Ausnehmung (74) in dem zweiten Bauteil (70) ragt und der in dem zweiten Bauteil (70) angeordnete zweite Bildsensor (50) oder dessen Platine (56) in eine Ausnehmung (64) in dem ersten Bauteil (60) ragt.

## Claims

1. A stereo endoscope (10) for capturing a first image intended for viewing with a first eye and a second image intended for viewing with a second eye, having:
a shaft (11) having a distal end region (13);
a first image sensor (30) in the distal end region (13) of the shaft (11) for capturing the first image;
a second image sensor (50) in the distal end region (13) of the shaft (11) for capturing the second image,
wherein both the photosensitive layer (32) of the first image sensor (30) and the photosensitive layer (52) of the second image sensor (50) are arranged parallel or substantially parallel to a longitudinal axis (18) of the distal end region (13),
wherein the first image sensor (30) and the second image sensor (50) are oriented in opposite or substantially opposite directions,
wherein the first image sensor (30) and the second image sensor (50) are offset relative to each other in a direction (86) orthogonal to a longitudinal axis (18) of the distal end region (13) of the shaft (11) and parallel to at least either a photosensitive layer (32) of the first image sensor (30) or a photosensitive layer (52) of the second image sensor (50),
**characterized in that** the first image sensor (30) has a first rectangular image detection region (32),
and an image (38) of a straight line parallel to a stereo base (16) of the stereo endoscope (10) in the photosensitive layer (32) of the first image sensor (30) is inclined with respect to straight edge portions (33, 34) of the rectangular image sensing region (32).

2. The stereo endoscope (10) according to the preceding claim, in which a photosensitive layer (32) of the first image sensor (30) and a photosensitive layer (52) of the second image sensor (50) in a direction (87) orthogonal to the photosensitive layers (32, 52) of both image sensors (30, 50) and orthogonal to a longitudinal axis (18) of the distal end region (13) of the shaft (11) are arranged offset relative to one another.

3. The stereo endoscope (10) according to any one of the preceding claims, in which the distal end region (13) of the shaft (11) has two identical or substantially identical components (60, 70), each comprising a cylindrical shape and a substantially semicircular cross-section.

4. The stereo endoscope (10) according to claim 3, in which an interface (67, 76) between the two components (60, 70) is neither parallel nor orthogonal to a stereo base (16) of the stereo endoscope (10).

5. The stereo endoscope (10) according to any one of claims 3 or 4, in which one of the components (60) has
a substantially closed lateral surface (62),
a recess (64) which is open towards the other component (70) and in which at least either the first image sensor (30) or the second image sensor (50) is arranged.

6. The stereo endoscope (10) according to any one of claims 3 to 5, in which the first image sensor (30) arranged in the first component (60) or its circuit board (36) projects into a recess (74) in the second component (70) and the second image sensor (50) arranged in the second component (70) or its circuit board (56) projects into a recess (64) in the first component (60).

7. The stereo endoscope (10) for capturing a first image intended for viewing with a first eye and a second image intended for viewing with a second eye, having:
a shaft (11) having a distal end region (13);
a first image sensor (30) in the distal end region (13) of the shaft (11) for capturing the first image;
a second image sensor (50) in the distal end region (13) of the shaft (11) for capturing the second image;
a first reflective surface (25) for reflecting light;
a second reflective surface (45) for reflecting light;
a first beam path (20) having a first straight portion (23) extending in the light propagation direction to the first reflective surface (25) and a second straight portion (26) extending from the first reflective surface (25) to the first image sensor (30);
a second beam path (40) having a first straight portion (43) extending in the light propagation direction to the second reflective surface (45) and a second straight portion (46) extending from the second reflective surface (45) to the second image sensor (50);
wherein the first straight portion (23) and the second straight portion (26) of the first beam path (20) are arranged in a first beam path plane (28),
wherein the first straight portion (43) and the second straight portion (46) of the second beam path (40) are arranged in a second beam path plane (48) which differs from the first beam path plane (28),
wherein a light propagation direction (27) of light in the second straight portion (26) of the first beam path (20) and a light propagation direction (47) of light in the second straight portion (46) of the second beam path (40) are opposite or substantially opposite,
**characterized in that** a photosensitive layer (32) of the first image sensor (30) is neither parallel nor orthogonal to a base plane in which the first straight portion (23) of the first beam path (20) and the first straight portion (43) of the second beam path (40) lie.

8. The stereo endoscope (10) according to claim 7, in which the second straight portion (26) of the first optical path (20) is arranged next to the second image sensor (50).

9. The stereo endoscope (10) according to any one of claims 7 or 8, in which the first image sensor (30) has a first rectangular image detection region (32), and an image (38) of a straight line parallel to a stereo base (16) of the stereo endoscope (10) in the photosensitive layer (32) of the first image sensor (30) is inclined with respect to straight edge portions (33, 34) of the rectangular image sensing region (32).

10. The stereo endoscope (10) according to any one of claims 7 to 9, in which the distal end region (13) of the shaft (11) has two identical or substantially identical components (60, 70), each comprising a cylindrical shape and a substantially semicircular cross-section.

11. The stereo endoscope (10) according to the preceding claim, in which each of the two components (60, 70) contains either the first beam path (20) or the second beam path (40) completely or to a large extent.

12. The stereo endoscope (10) according to any one of claims 10 and 11, in which an interface (67, 76) between the two components (60, 70) is neither parallel nor orthogonal to a stereo base (16) of the stereo endoscope (10).

13. The stereo endoscope (10) according to any one of claims 10 to 12, in which one of the components (60) has
a substantially closed lateral surface (62),
a recess (64) which is open towards the other component (70) and in which at least either the first image sensor (30) or the second image sensor (50) is arranged.

14. The stereo endoscope (10) according to any one of claims 10 to 13, in which the first image sensor (30) arranged in the first component (60) or its circuit board (36) projects into a recess (74) in the second component (70) and the second image sensor (50) arranged in the second component (70) or its circuit board (56) projects into a recess (64) in the first component (60).

## Revendications

1. Stéréo-endoscope (10) pour capturer une première image destinée à être visualisée avec un premier oeil et une seconde image destinée à être visualisée avec un second oeil, ayant :
une tige (11) ayant une région d'extrémité distale (13) ;
un premier capteur d'image (30) dans la région d'extrémité distale (13) de la tige (11) pour capturer la première image ;
un second capteur d'image (50) dans la région d'extrémité distale (13) de la tige (11) pour capturer la seconde image,
dans lequel à la fois la couche photosensible (32) du premier capteur d'image (30) et la couche photosensible (52) du second capteur d'image (50) sont disposées parallèle ou sensiblement parallèle à un axe longitudinal (18) de la région d'extrémité distale (13),
dans lequel le premier capteur d'image (30) et le second capteur d'image (50) sont orientés dans des directions opposées ou sensiblement opposées,
dans lequel le premier capteur d'image (30) et le second capteur d'image (50) sont décalés l'un par rapport à l'autre dans une direction (86) orthogonale à un axe longitudinal (18) de la région d'extrémité distale (13) de la tige (11) et parallèlement à au moins soit une couche photosensible (32) du premier capteur d'image (30) soit une couche photosensible (52) du second capteur d'image (50), **caractérisé en ce que** le premier capteur d'image (30) a une première région de détection d'image rectangulaire (32),
et une image (38) d'une ligne droite parallèle à une base stéréo (16) du stéréo-endoscope (10) dans la couche photosensible (32) du premier capteur d'image (30) est inclinée par rapport aux parties de bord droit (33, 34) de la région de détection d'image rectangulaire (32).

2. Stéréo-endoscope (10) selon la revendication précédente, dans lequel une couche photosensible (32) du premier capteur d'image (30) et une couche photosensible (52) du second capteur d'image (50) dans une direction (87) orthogonale aux couches photosensibles (32, 52) des deux capteurs d'image des capteurs (30, 50) et orthogonaux à un axe longitudinal (18) de la zone d'extrémité distale (13) de la tige (11) sont disposées décalées l'une par rapport à l'autre.

3. Stéréo-endoscope (10) selon l'une quelconque des revendications précédentes, dans lequel la région d'extrémité distale (13) de la tige (11) a deux composants identiques ou sensiblement identiques (60, 70), chacun comprenant une forme cylindrique et une section transversale sensiblement semi-circulaire.

4. Stéréo-endoscope (10) selon la revendication 3, dans lequel une interface (67, 76) entre les deux composants (60, 70) n'est ni parallèle ni orthogonale à une base stéréo (16) du stéréo endoscope (10).

5. Stéréo-endoscope (10) selon l'une quelconque des revendications 3 ou 4, dans lequel l'un des composants (60) a
une surface latérale sensiblement fermée (62),
un évidement (64) qui est ouvert vers l'autre composant (70) et dans lequel au moins soit le premier capteur d'image (30) soit le second capteur d'image (50) est agencé.

6. Stéréo-endoscope (10) selon l'une quelconque des revendications 3 à 5, dans lequel le premier capteur d'image (30) agencé dans le premier composant (60) ou la carte de circuit (36) de celui-ci fait saillie dans un évidement (74) du second composant (70) et le second capteur d'image (50) agencé dans le second composant (70) ou la carte de circuit (56) de celui-ci fait saillie dans un évidement (64) du premier composant (60).

7. Le stéréo-endoscope (10) pour capturer une première image destinée à être visualisée avec un premier oeil et une seconde image destinée à être visualisée avec un second oeil, ayant :
une tige (11) ayant une région d'extrémité distale (13) ;
un premier capteur d'image (30) dans la région d'extrémité distale (13) de la tige (11) pour capturer la première image ;
un second capteur d'image (50) dans la région d'extrémité distale (13) de la tige (11) pour capturer la seconde image ;
une première surface réfléchissante (25) pour réfléchir la lumière ;
une seconde surface réfléchissante (45) pour réfléchir la lumière ;
un premier trajet de faisceau (20) ayant une première partie droite (23) s'étendant dans la direction de propagation de la lumière vers la première surface réfléchissante (25) et une seconde partie droite (26) s'étendant de la première surface réfléchissante (25) vers le premier capteur d'image (30) ;
un second trajet de faisceau (40) ayant une première partie droite (43) s'étendant dans la direction de propagation de la lumière vers la seconde surface réfléchissante (45) et une seconde partie droite (46) s'étendant de la seconde surface réfléchissante (45) vers le second capteur d'image (50) ;
dans lequel la première partie droite (23) et la seconde partie droite (26) du premier trajet de faisceau (20) sont agencées dans un premier plan de trajet de faisceau (28),
dans lequel la première partie droite (43) et la seconde partie droite (46) du second trajet de faisceau (40) sont agencées dans un second plan de trajet de faisceau (48) qui diffère du premier plan de trajet de faisceau (28),
dans lequel une direction de propagation de la lumière (27) de la lumière dans la seconde partie droite (26) du premier trajet de faisceau (20) et une direction de propagation de la lumière (47) de la lumière dans la seconde partie droite (46) du second trajet de faisceau (40) sont opposés ou sensiblement opposés,
**caractérisé en ce qu'**une couche photosensible (32) du premier capteur d'image (30) n'est ni parallèle ni orthogonale à un plan de base dans lequel la première partie droite (23) du premier trajet de faisceau (20) et la première partie droite (43) du second trajet de faisceau (40).

8. Stéréo-endoscope (10) selon la revendication 7, dans lequel la seconde partie droite (26) du premier trajet optique (20) est agencée à côté du second capteur d'image (50).

9. Stéréo-endoscope (10) selon l'une quelconque des revendications 7 ou 8, dans lequel
le premier capteur d'image (30) a une première région de détection d'image rectangulaire (32), et une image (38) d'une ligne droite parallèle à une base stéréo (16) du stéréo-endoscope (10) dans la couche photosensible (32) du premier capteur d'image (30) est inclinée par rapport aux parties de bord droit (33, 34) de la région de détection d'image rectangulaire (32).

10. Stéréo-endoscope (10) selon l'une quelconque des revendications 7 à 9, dans lequel la région d'extrémité distale (13) de la tige (11) a deux composants identiques ou sensiblement identiques (60, 70), chacun comprenant une forme cylindrique et une section transversale sensiblement semi-circulaire.

11. Stéréo-endoscope (10) selon la revendication précédente, dans lequel chacun des deux composants (60, 70) contient soit le premier trajet de faisceau (20) soit le second trajet de faisceau (40) complètement ou dans une large mesure.

12. Stéréo-endoscope (10) selon l'une quelconque des revendications 10 et 11, dans lequel une interface (67, 76) entre les deux composants (60, 70) n'est ni parallèle ni orthogonale à une base stéréo (16) du stéréo endoscope (10).

13. Stéréo-endoscope (10) selon l'une quelconque des revendications 10 à 12, dans lequel l'un des composants (60) a
une surface latérale sensiblement fermée (62),
un évidement (64) qui est ouvert vers l'autre composant (70) et dans lequel au moins soit le premier capteur d'image (30) soit le second capteur d'image (50) est agencé.

14. Stéréo-endoscope (10) selon l'une quelconque des revendications 10 à 13, dans lequel le premier capteur d'image (30) agencé dans le premier composant (60) ou la carte de circuit (36) de celui-ci fait saillie dans un évidement (74) du second composant (70) et le second capteur d'image (50) agencé dans le second composant (70) ou la carte de circuit (56) de celui-ci fait saillie dans un évidement (64) du premier composant (60).
